# EUROPÄISCHE PATENTSCHRIFT

(11) **EP 1 255 559 B1**
(45) Veröffentlichungstag und Bekanntmachung des Hinweises auf die Patenterteilung: **28.04.2004**
(21) Anmeldenummer: 01913667.0
(22) Anmeldetag: 16.02.2001
(51) Int. Cl.: A61K 38/55, A61K 48/00, A61K 38/16, A61K 38/17, A61K 38/43, A61K 39/395, A61K 31/7088, A61P 9/00

(54) **VERWENDUNG VON INHIBITOREN VON CASPASE-3 ODER DER CASPASE-AKTIVIERTEN DESOXYRIBONUCLEASE (CAD) ZUR BEHANDLUNG VON HERZERKRANKUNGEN**
USE OF INHIBITORS OF CASPASE-3 OR CASPASE-ACTIVATED DESOXYRIBONUCLEASE (CAD) FOR TREATING CARDIAC DISEASE
UTILISATION D'INHIBITEURS DE LA CASPASE-3 OU DE LA DESOXYRIBONUCLEASE ACTIVEE PAR LA CASPASE (CAD) POUR LE TRAITEMENT DE MALADIES CARDIAQUES

(30) Priorität: 16.02.2000 DE 10006889
(43) Veröffentlichungstag der Anmeldung: 13.11.2002
(73) Patentinhaber: Procorde GmbH, 82152 Martinsried (DE)
(72) Erfinder: LAUGWITZ, Karl-Ludwig, La Jolla, CA 92037 (US); MORETTI, Alessandra, La Jolla, CA 92037 (US); UNGERER, Martin, 80799 München (DE)
(74) Vertreter: Hartz, Nikolai F., Dr.
(86) Internationale Anmeldenummer: PCT/DE2001/000616
(87) Internationale Veröffentlichungsnummer: WO 2001/060400

(56) Entgegenhaltungen:
- WO-A-96/33268
- WO-A-99/10501
- WO-A-99/18781
- WO-A-99/54482
- DATABASE WPI Section Ch, Week 199947 Derwent Publications Ltd., London, GB; Class B04, AN 1999-554024 XP002177298 & JP 11 239495 A (ZH OSAKA BIOSCIENCE KENKYUSHO), 7. September 1999 (1999-09-07)
- ELDADAH BASIL A ET AL: "Ribozyme-mediated inhibition of caspase-3 protects cerebellar granule cells from apoptosis induced by serum-potassium deprivation." JOURNAL OF NEUROSCIENCE, Bd. 20, Nr. 1, 1. Januar 2000 (2000-01-01), Seiten 179-186, XP002177296 ISSN: 0270-6474
- WEIG HANS-JOERG ET AL: "Adenoviral overexpression of p35 preserves contractility in pacing induced heart failure in vivo." CIRCULATION, Bd. 102, Nr. 18 Supplement, 31. Oktober 2000 (2000-10-31), Seite II.165 XP001024161 Abstracts from Scientific Sessions 2000;New Orleans, Louisiana, USA; November 12-15, 2000 ISSN: 0009-7322
- LAUGWITZ KARL-LUDWIG ET AL: "Blocking caspase-3-activated apoptosis mechanism prevents progression of heart failure." CIRCULATION, Bd. 102, Nr. 18 Supplement, 31. Oktober 2000 (2000-10-31), Seite II.11 XP001024162 Abstracts from Scientific Sessions 2000;New Orleans, Louisiana, USA; November 12-15, 2000 ISSN: 0009-7322

## Beschreibung

Die vorliegende Erfindung betrifft Screeningverfahren zum Auffinden eines Inhibitors von Caspase-3 oder der Caspase-aktivierten Desoxyribonuclease (CAD) zur Prävention oder Behandlung von Herzerkrankungen, wobei es sich bei diesen Herzerkrankungen vorzugsweise um eine Insuffizienz der linken Herzkammer handelt. Ein Beispiel für einen Inhibitor ist ICAD. Die Verabreichung des Inhibitors kann über einen Adenovirus-Vektor, der das diesen Inhibitor codierende Gen in exprimierbarer Form enthält erfolgen. Die vorliegende Erfindung betrifft Verfahren zur Identifizierung von Inhibitoren für die vorstehende therapeutische Anwendung, d.h. Verbindungen, die Caspase-3 oder CAD bzw. die Expression der diese Verbindungen codierenden Gene hemmen.

Bei der Apoptose handelt es sich um eine genetisch gesteuerte Form des Zelltods, der für die normale Entwicklung und das physiologische Gleichgewicht von Organismen essentiell ist. Viele degenerative Erkrankungen stehen mit abnorm hohen Apoptose-Spiegeln in Zusammenhang. Der programmierte Zelltod der Kardiomyocyten konnte bei einer Reihe von kardiovaskulären Erkrankungen nachgewiesen werden, z.B. beim Myokardinfarkt und der Stauungsherzinsuffizienz (CHF). Apoptose könnte die Folge einer verlängerten Wachstumsstimulation adulter Kardiomyocyten sein, die - als terminal differenziertes Gewebe - nicht mehr zur Teilung befähigt sind. Als Kompensation der chronisch veränderten hämodynamischen Erfordernisse hinsichtlich des versagenden Herzmuskels tritt eine hypertrophe Reaktion ein und diese wird durch die systemische und lokale Hochregulation des adrenergischen Systems und des Renin/Angiotensin-Systems und durch verschiedene Cytokine vermittelt. Allerdings wurden bisher keine Therapien der vorstehend genannten Herzerkrankungen angewandt, die darauf abzielten, die Apoptose zu hemmen. Die bisherigen pharmakologischen Interventionen bei diesen Herzerkrankungen, die vornehmlich auf einer β-adrenergischen Blockierung beruhen, sind nicht in allen Fällen erfolgreich bzw. zeigen oft eine nicht ausreichende Wirkung.

WO 99 10501 offenbart die Rolle von Caspase-aktivierter Desoxyribonuclease (CAD = CPAN = DFF40) in Zell-Apoptose und ebenfalls deren Regulierung mittels ICAD (= DFF45) (Seite 8 2. und 3. Absatz). Inhibierung von CAD durch ICAD Protein (Beispiel 8), durch Transfektion mit dem mutierten ICAD Gen, dessen Produkt nicht von Caspase-3 gespalten werden kann (Beispiel 9), durch CAD-antisense Stränge (Seite 10, 3. Absatz, Ansprüche 36, 39) und durch CAD- gerichtete mono- und polyklonale Antikörper (Seite 13 4. Absatz, Anspruch 35) wird erwähnt. Als Expressionsvektoren für die Transfektion wird Adenovirus nicht genannt. Diese Apoptose-Inhibitoren werden als nützlich für die Behandlung von Herzanfall oder Gehirnschlag gesehen, da diese Pathologien Zellschäden durch Apoptose induzieren (Seite 12 letzter Absatz). Ein Assay zur Identifizierung von CAD Inhibitoren, der darin besteht, dass Testsubstanzen mit CAD in Kontakt gebracht werden und gleichzeitig die Fähigkeit von CAD gemessen wird, DNA zu fragmentieren, wird auch beschrieben und beansprucht (Seite 12, 4. Absatz, Ansprüche 24 - 27, 41).

WO 96 33268 beschreibt eine Methode zur Identifikation von Substanzen, die Caspase-3 (= Apopain = CPP32) in ihrer Aktivität modulieren. Die Methode besteht in der Mischung von Caspase-3 mit der zu testenden Substanz und der Messung der Caspase-3 Aktivität (Seite 4, Zeilen 17 - 20, Anspruch 4). Weiter werden Modulatoren der Aktivität von Caspase-3 sowie Caspase-3 Antisens- Moleküle zur Behandlung von Kardiovaskulären Verletzungen (Seite 4 Zeile 25 - Seite 5 Zeile 3, Seite 6 Zeilen 9 - 27) erwähnt. Die Antisens-Moleküle können DNA oder RNA sein und mittels Mikroinjektion, Liposom-Einkapselung oder Expressionsvektoren in Zellen eingeführt werden (Seite 14 Zeilen 1 - 10). Adenovirus als Vektor wird für den Transfer des Caspase-3 Gens wird angeführt (Seite 14 Zeilen 12 - 16).

Somit liegt der Erfindung im wesentlichen das technische Problem zugrunde, alternative Mittel bereitzustellen, die für die pharmakologische Therapie von Herzerkrankungen von Nutzen sind.

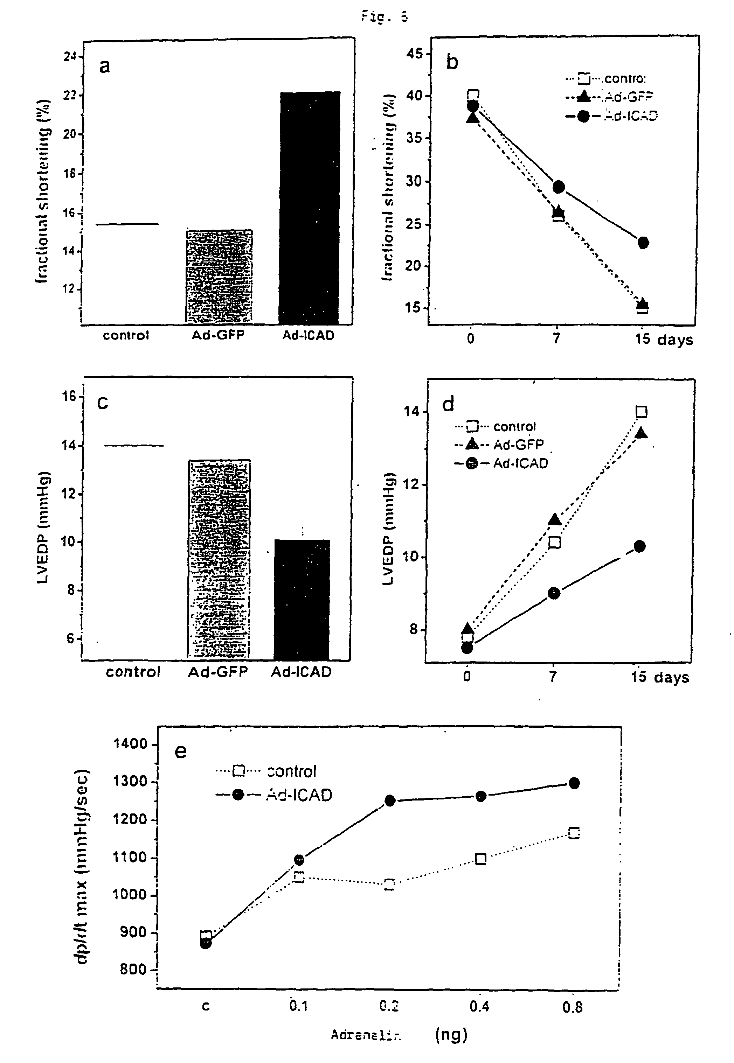

Die Lösung dieses technischen Problems wurde durch die Bereitstellung der in den Patentansprüchen gekennzeichneten Ausführungsformen erreicht. Die Apoptose des Herzmuskels ist eine höchst komplex regulierte Zell-Selbstmordmaschine, bei der zwei Haupt-Signalwege zur Aktivierung der Caspase-Familie und zur Förderung der Translokation von Caspase-aktivierter DNase (CAD) in den Nucleus führen: (a) "Todesrezeptor"-Signalgebung (z.B. Fas-, TNF- und DR3-DR6-Rezeptoren) und (b) Freisetzung von Cytochrom b aus den Mitochondrien und im Anschluß daran Transaktivierung von Procaspase 9 durch Apaf. Die Caspase-Familie der Cystein-Proteasen reguliert den Eintritt der Apoptose bei Säugern. Caspase-3 stellt das Schlüsselenzym dar und dieses spaltet stromabwärts gelegene Ziele, die an der Expression des apoptotischen Phänotyps beteiligt sind, z.B. Gelsolin, PAK2, nucleäre Lamine und insbesondere die hemmende Untereinheit des DNA-Fragmentierungsfaktors (ICAD). Das wichtigste biochemische Merkmal der Apoptose ist die Spaltung chromosomaler DNA in nucleosomale Einheiten, was das finale Ereignis bei der Apoptose zu sein scheint. Die für den DNA-Abbau bei der Apoptose verantwortliche Nuclease ist CAD. CAD wird als ein Komplex mit ICAD zur Hemmung ihrer DNase-Aktivität produziert. Die stromaufwärts durch apoptotische Stimuli aktivierte Caspase-3 spaltet ICAD, was es dann CAD erlaubt, in den Nucleus einzutreten und chromosomale DNA abzubauen. Während der Untersuchungen, die zu der vorliegenden Erfindung führten, wurde festgestellt, daß die Aktivitäten von Caspase-3 und CAD, den zwei Schlüsselmolekülen im Prozeß der myokardialen Apoptose in einem Tiermodell der CHF, bei dem die Veränderungen auf der hämodynamischen und biochemischen Ebene denen bei der entsprechenden Erkrankung des menschlichen Herzens gleichen, erhöht sind. Die Stimulierung von Caspase-3 führt letztendlich zur Aktivierung von CAD und somit sind beide Enzyme für das Fortschreiten der Herzinsuffizienz erforderlich. Es konnte in den Untersuchungen gezeigt werden, daß durch Adenovirus-vermittelte Expression von p35 oder ICAD in Kardiomyocyten Caspase-3- und CAD-Aktivität signifikant gehemmt werden konnte. Die Hemmung dieser Schlüsselenzyme wurde auch durch eine verringerte DNA-Fragmentierung durch ICAD und, wenn auch in geringerem Ausmaß, durch p35 widergespiegelt. Da die ICAD-Expression zur Hemmung der DNA-Fragmentierung in vitro eindeutig wirksamer war, wurde die Expression des ICAD codierenden Transgens auch in vivo an Kaninchen mit Herzinsuffizienz untersucht. Während die Infektion von Kaninchen-Myokard mit rekombinanten Kontroll-Adenoviren keine Auswirkung auf die Kardiomyocyten-Apoptose hatte, wurde die hämodynamische Funktion von ICAD-exprimierenden Herzen mit Insuffizienz zumindest partiell wieder hergestellt (Fig. 6). So konnten eine verbesserte Kontraktionsfähigkeit und ein verringerter enddiastolischer Druck der linken Kammer bei einer Verringerung der Caspase-3-induzierten DNA-Fragmentierung beobachtet werden. Es wird davon ausgegangen, daß bei den vorstehenden Krankheitsbildern die meisten Kardiomyocyten in einem prae-apoptotischen Zustand sind mit erhöhten Aktivitäten von an der Apoptose beteiligten Enzymen, was eine Bereitschaft dieser Zellen zur Apoptose ausdrückt, jedoch noch nicht die tatsächliche Ausführung dieses Prozesses anzeigt. Die vorliegenden Ergebnisse zeigen jedenfalls, daß myokardiale Apoptose zum Fortschreiten der Herzinsuffizienz beiträgt und daß das Verhindern der Apoptose bei Kardiomyocyten eine nützliche Funktion hat, d.h., das Verhindern der Apoptose stellt eindeutig ein attraktives Ziel für eine therapeutische Intervention dar. Anhand der Ergebnisse mit insuffizienten, mit Ad-p35 oder Ad-ICAD infizierten Herzen konnte auch gezeigt werden, daß ein antiapoptotischer Ansatz bei Herzinsuffizienz nicht nur die nukleäre DNA-Fragmentierung verhindert, sondern außerdem die Sarkomerorganisation aufrecht erhält und damit die Kontraktionskraft der noch lebenden Herzmuskelzellen verbessert. Somit kann für eine therapeutische Intervention bei den vorstehend diskutierten Erkrankungen des Herzens, die mit einer Apoptose von Kardiomyocyten in Zusammenhang stehen, die Verabreichung eines Faktors, der die Aktivität von CAD oder Caspase-3 hemmt, oder des diesen Faktor codierenden Gens nützlich sein. Andererseits kann auch die Möglichkeit der Hemmung der Expression des CAD oder Caspase-3 codierenden Gens therapeutisch sinnvoll sein. Eine solche Hemmung kann somit auf verschiedenen Ebenen erfolgen, beispielsweise auf genetischer Ebene ("Knock out", Hemmung der Translation durch Antisense-RNAs oder Ribozyme) oder Proteinebene (über CAD bzw. Caspase-3 hemmende Antikörper, ICAD etc.)

Gegenstand der vorliegenden Erfindung ist somit ein Screeningverfahren zum Auffinden eines Inhibitors von Caspase-3 oder der Caspase-aktivierten Desoxyribonuclease (CAD) zur Prävention oder Behandlung von Herzerkrankungen, vorzugsweise einer Herzinsuffizienz, insbesondere einer Insuffizienz der linken Herzkammer.

Der Inhibitor ist beispielsweise eine Verbindung, die die Expression des CAD oder Caspase-3 codierenden Gens hemmt, z.B ein Ribozym oder eine Antisense-RNA. Da die gesamte Nucleinsäuresequenz des CAD bzw. Caspase-3 codierenden Gens bekannt ist, kann der Fachmann mittels Routineverfahren solche Verbindungen identifizieren und, z.B. mittels der in den nachstehenden Beispielen beschriebenen Vorgehensweisen deren Wirkung testen.

Daher betrifft eine stärker bevorzugte Ausführungsform der vorliegenden Erfindung ein Screeningverfahren zum Auffinden einer Antisense-RNA, die dadurch gekennzeichnet ist, daß sie zu der von dem CAD oder Caspase-3 codierenden Gen transkribierten mRNA oder einem Teil davon, vorzugsweise dem codierenden Bereich, komplementär ist und an diese mRNA spezifisch binden kann, wodurch die Synthese von CAD oder Caspase-3 verringert oder gehemmt wird. Eine weitere stärker bevorzugte Ausführungsform der vorliegenden Erfindung betrifft ein Screeningverfahren zum Auffinden eines Ribozyms, das dadurch gekennzeichnet ist, daß es zu der von dem CAD oder Caspase-3 codierenden Gen transkribierten mRNA oder einem Teil davon komplementär ist und an diese mRNA spezifisch binden und diese spalten kann, wodurch die Synthese von CAD oder Caspase-3 verringert oder gehemmt wird. Der Fachmann ist in der Lage, ausgehend von den offenbarten CAD- bzw. Caspase-3-Sequenzen, geeignete Antisense-RNAs herzustellen und anzuwenden. Geeignete Vorgehensweisen sind beispielsweise in EB-B1 0 223 399 oder EP-A1 0 458 beschrieben. Ribozyme sind RNA-Enzyme und bestehen aus einem einzelnen RNA-Strang. Diese können andere RNAs intermolekular spalten, beispielsweise die von den CAD bzw. Caspase-3 codierenden Sequenzen transkribierten mRNAs. Diese Ribozyme müssen prinzipiell über zwei Domänen verfügen, (1) eine katalytische Domäne und (2) eine Domäne, die zu der Ziel-RNA komplementär ist und an diese binden kann, was die Voraussetzung für eine Spaltung der Ziel-RNA ist. Ausgehend von in der Literatur beschriebenen Vorgehensweisen ist es inzwischen möglich, spezifische Ribozyme zu konstruieren, die eine gewünschte RNA an einer bestimmten, vorgewählten Stelle schneiden (siehe beispielsweise Tanner et al., in: Antisense Research and Applications, CRC Press, Inc. (1993), 415-426).

In einer weiteren bevorzugten Ausführungsform des erfindungsgemäßen Screeningverfahrens zum Auffinden von Caspase-3- oder CAD-Inhibitoren ist der Inhibitor das in den nachfolgenden Beispielen beschriebene ICAD oder Baculovirus-p35.

In einer weiteren bevorzugten Ausführungsform des erfindungsgemäßen Screeningverfahrens zum Auffinden von Caspase-3- oder CAP-Inhibitoren ist der Inhibitor ein Antikörper, der an Caspase-3 oder CAP bindet, oder ein Fragment davon. Diese Antikörper können monoclonale, polyclonale oder synthetische Antikörper sein oder Fragmente davon. In diesem Zusammenhang bedeutet der Begriff "Fragment" alle Teile des monoclonalen Antikörpers (z.B. Fab-, Fv- oder "single chain Fv"-Fragmente), welche die gleiche Epitopspezifität wie der vollständige Antikörper aufweisen. Die Herstellung solcher Fragmente ist dem Fachmann bekannt. Vorzugsweise handelt es sich bei den erfindungsgemäßen Antikörpern um monoclonale Antikörper. Die erfindungsgemäßen Antikörper können gemäß Standardverfahren hergestellt werden, wobei vorzugsweise das Caspase-3- bzw. CAP-Protein oder ein synthetisches Fragment davon als Immunogen dienen. Verfahren zur Gewinnung monoclonaler Antikörper sind dem Fachmann bekannt. Der monoclonale Antikörper kann ein aus einem Tier (z.B. Maus) stammender Antikörper, ein humanisierter Antikörper oder ein chimärer Antikörper oder ein Fragment davon sein. Chimäre, menschlichen Antikörper ähnelnde oder humanisierte Antikörper besitzen eine herabgesetzte potentielle Antigenität, jedoch ist ihre Affinität gegenüber dem Ziel nicht herabgesetzt. Die Herstellung von chimären und humanisierten Antikörpern bzw. von den menschlichen Antikörpern ähnelnden Antikörpern wurde ausführlich beschrieben (siehe beispielsweise Queen et al., Proc. Natl. Acad. Sci. USA 86 (1989), 10029, und Verhoeyan et al., Science 239 (1988), 1534). Humanisierte Immunglobuline weisen variable Grundgerüstbereiche auf, die im wesentlichen von einem humanen Immunglobulin stammen (mit der Bezeichnung Akzeptor-Immunglobulin) und die Komplementarität der determinierenden Bereiche, die im wesentlichen von einem nicht-menschlichen Immunglobulin (z.B. von der Maus) stammen (mit der Bezeichnung Donor-Immunglobulin). Die (der) konstante(n) Bereich(e) stammt (stammen), falls vorhanden, auch im wesentlichen von einem menschlichen Immunglobulin. Bei der Verabreichung an menschliche Patienten bieten humanisierte (sowie die menschlichen) Antikörper eine Reihe von Vorteilen gegenüber Antikörpern von Mäusen oder anderen Spezies: (a) das menschliche Immunsystem sollte das Grundgerüst oder den konstanten Bereich des humanisierten Antikörpers nicht als fremd erkennen und daher sollte die Antikörperantwort gegen einen solchen injizierten' Antikörper geringer ausfallen als gegen einen vollständig fremden Maus-Antikörper oder einen partiell fremden chimären Antikörper; (b) da der Effektorbereich des humanisierten Antikörpers menschlich ist, interagiert er besser mit anderen Teilen des menschlichen Immunsystems, und (c) injizierte humanisierte Antikörper weisen eine Halbwertszeit auf, die im wesentlichen zu der von natürlich vorkommenden menschlichen Antikörpern äquivalent ist, was es erlaubt, kleinere und weniger häufige Dosen im Vergleich zu Antikörpern anderer Spezies zu verabreichen.

Die vorstehend diskutierten Inhibitoren werden vorzugsweise nicht selbst verabreicht, sondern über eine Gentherapie, d.h. die diese Inhibitoren (z.B. Ribozyme, Antisense-RNAs, Antikörper, ICAD, Baculovirus-p35) codierenden DNA-Sequenzen werden, vorzugsweise in einen Expressionsvektor inseriert, zu dem Zielorgan gebracht. Somit umfaßt die vorliegende Erfindung auch diese Inhibitoren codierende DNA-Sequenzen enthaltende Expressionsvektoren. Die Bezeichnung "Vektor" bezieht sich auf ein Plasmid (pUC18, pBR322, pBlueScript etc.), auf ein Virus oder ein anderes geeignetes Vehikel. Diese DNA-Sequenzen sind im Vektor mit regulatorischen Elementen funktionell verknüpft, die deren Expression in prokaryotischen oder eukaryotischen Wirtszellen erlauben. Solche Vektoren enthalten neben den regulatorischen Elementen, beispielsweise einem Promotor, typischerweise einen Replikationsursprung und spezifische Gene, die die phänotypische Selektion einer transformierten Wirtszelle erlauben. Zu den regulatorischen Elementen für die Expression in Prokaryoten, beispielsweise E.coli, zählen der lac-,trp-Promotor oder T7-Promotor, und für die Expression in Eukaryoten der AOX1- oder GAL1-Promotor in Hefe und der CMV- ,SV40-,RVS-40-Promotor, CMV- oder SV40-Enhancer für die Expression in tierischen Zellen. Geeignete regulatorische Sequenzen sind außerdem beschrieben in Goeddel: Gene Expression Technology: Methods in Enzymology 185, Academic Press, San Diego, CA (1990). Weitere Beispiele für geeignete Promotoren sind der Metallothionein I- und der Polyhedrin-Promotor. Zu geeigneten Expressionsvektoren für E.coli zählen beispielsweise pGEMEX, pUC-Derivate, pGEX-2T, pET3b und pQE-8. Zu den für die Expression in Hefe geeigneten Vektoren zählen pY100 und Ycpad1, für die Expression in Säugerzellen pMSXND, pKCR, pEFBOS, cDM8 und pCEV4 sowie von pcDNAI/amp, pcDNAI/neo, pRc/CMV, pSV2gpt, pSV2neo, pSV2-dhfr, pTk2, pRSVneo, pMSG, pSVT7, pko-neo und pHyg stammende Vektoren.

Vorzugsweise werden die vorstehend beschriebenen DNA-Sequenzen in einen für die Gentherapie geeigneten Vektor inseriert, beispielsweise unter Kontrolle eines gewebespezifischen Promotors, und in die Zellen eingeschleust. In einer bevorzugten Ausführungsform ist der die vorstehend beschriebenen DNA-Sequenzen enthaltende Vektor ein Virus, beispielsweise ein Adenovirus, Vaccinia-Virus oder Retrovirus. Beispiele für geeignete Retroviren sind MoMuLV, HaMuSV, MuMTV, RSV oder GaLV. Besonders bevorzugt sind Adenoviren, insbesondere solche mit E1 und/oder E3-Mutationen (Deletionen), die zusätzlich auch eine E4-Mutation (Deletion) aufweisen können, oder "gutless" Adenoviren. Für die Gentherapie geeignete Vektoren sind außerdem in WO 93/04701, WO 92/22635, WO 92/20316, WO 92/19749 und WO 92/06180 offenbart. Für Zwecke der Gentherapie können die erfindungsgemäßen DNA-Sequenzen auch in Form von kolloidalen Dispersionen zu den Zielzellen transportiert werden. Dazu zählen beispielsweise Liposomen oder Lipoplexe (Mannino et al., Biotechniques 6 (1988), 682).

Allgemeine, auf dem Fachgebiet bekannte Verfahren können zur Konstruktion von Expressionsvektoren, die diese DNA-Sequenzen und geeignete Kontrollsequenzen enthalten, verwendet werden. Zu diesen Verfahren zählen beispielsweise in vitro-Rekombinationstechniken, synthetische Verfahren, sowie in vivo-Rekombinationsverfahren, wie sie in gängigen Lehrbüchern beschrieben sind.

Die vorstehenden Verbindungen bzw. die diese codierenden DNA-Sequenzen oder Vektoren werden gegebenenfalls mit einem pharmazeutisch verträglichen Träger verabreicht. Geeignete Träger und die Formulierung derartiger Arzneimittel sind dem Fachmann bekannt. Zu geeigneten Trägern zählen beispielsweise Phosphatgepufferte Kochsalzlösungen, Wasser, Emulsionen, beispielsweise Öl/Wasser-Emulsionen, Netzmittel, sterile Lösungen etc. Die geeignete Dosierung wird von dem behandelnden Arzt bestimmt und hängt von verschiedenen Faktoren ab, beispielsweise von dem Alter, dem Geschlecht, dem Gewicht des Patienten, der Art und dem Stadium der Herzerkrankung, der Art der Verabreichung etc.

Die vorliegende Erfindung betrifft ein Verfahren zur Identifizierung einer Verbindung, die als Inhibitor bei den vorstehend beschriebenen therapeutischen Vorgehensweisen wirksam ist, wobei das Verfahren (a) das Inkontaktbringen der eventuell als Inhibitor geeigneten Testverbindung mit Caspase-3 oder CAD bzw. mit dem Caspase-3 oder CAD codierenden Gen und (b) die Bestimmung der noch verbleibenden Caspase-3- bzw. CAD-Aktivität bzw. der Erniedrigung der Genexpression umfaßt, vorzugsweise im Vergleich zu einem Kontroll-Assay, in dem die Testverbindung nicht vorhanden ist. Eine erniedrigte oder vollständig gehemmte Enzymaktivität bzw. Genexpression zeigt an, daß die Testverbindung als Inhibitor wirksom ist. Geeignete Assays sind dem Fachmann bekannt und z.B. auch in den nachstehenden Beispielen beschrieben. Dieses Verfahren kann auch in einem zellulären Assay durchgeführt werden. Bei den Testverbindungen kann es sich um sehr unterschiedliche Verbindungen handeln, sowohl um natürlich vorkommende als auch synthetische, organische und anorganische Verbindungen, sowie um Polymere (z.B. Oligopeptide, Polypeptide, Oligonucleotide und Polynucleotide) sowie kleine Moleküle, Antikörper, Zucker, Fettsäuren, Nucleotide und Nucleotid-Analoge, Analoge von natürlich vorkommenden Strukturen (z.B. Peptid-"Imitatoren", Nucleinsäureanaloge etc.) und zahlreiche weitere Verbindungen. Darüber hinaus kann eine große Anzahl von möglicherweise nützlichen, die Kardiomyocyten-Apoptose hemmenden Verbindungen in Extrakten von natürlichen Produkten als Ausgangsmaterial gescreent werden. Solche Extrakte können aus einer großen Anzahl von Quellen stammen, beispielsweise den Spezies Pilze, Actinomyceten, Algen, Insekten, Protozoen, Pflanzen und Bakterien. Die Extrakte, die Aktivität zeigen, können dann zur Isolierung des aktiven Moleküls analysiert werden. Siehe beispielsweise Turner, J. Ethnopharmacol. 51 (1-3) (1996), 39-43 und Suh, Anticancer Res. 15 (1995) 233-239. Grundsätzlich geeignete Assay-Formate für die Identifizierung von Testverbindungen, die die Expression oder Aktivität von CAD oder Caspase-3 beeinflussen, sind in der biotechnologischen und pharmazeutischen Industrie gut bekannt und für den Fachmann sind zusätzliche Assays und Variationen dieser Assays offensichtlich. Änderungen im Expressionsspiegel von Caspase-3 oder CAD können unter Verwendung von dem Fachmann gut bekannten Verfahren untersucht werden. Dazu zählt die Überwachung der mRNA-Konzentration (z.B. unter Verwendung geeigneter Sonden oder Primer), Immunassays hinsichtlich der Proteinkonzentration RNAse-Schutzassays, Amplifikationsassays oder jedes andere für einen Nachweis geeignete Mittel, das auf dem Fachgebiet bekannt ist.

Das Suchen nach Verbindungen, die für eine Therapie über die Verhinderung der Kariomyocyten-Apoptose wirksam sind, kann auch in großem Maßstab erfolgen, beispielsweise dadurch, daß eine sehr hohe Anzahl von Kandidaten-Verbindungen in Substanzbibliotheken gescreent wird, wobei die Substanzbibliotheken synthetische oder natürliche Moleküle enthalten können. Jedenfalls können die Herstellung und das simultane Screenen von großen Banken aus synthetischen Molekülen mittels gut bekannter Verfahren der kombinatorischen Chemie ausgeführt werden, siehe beispielsweise van Breemen, Anal. Chem. 69 (1997), 2159-2164 und Lam, Anticancer Drug Des. 12 (1997), 145-167. Das erfindungsgemäße Verfahren kann auch als Screening mit hohem Durchsatz ("high throughput screening") stark beschleunigt werden. Die hier beschriebenen Assays können zur Verwendung in einem solchen Verfahren entsprechend modifiziert werden. Es ist für den Fachmann offensichtlich, daß für diesen Zweck zahlreiche Verfahren zur Verfügung stehen.

### Kurze Beschreibung der Figuren:

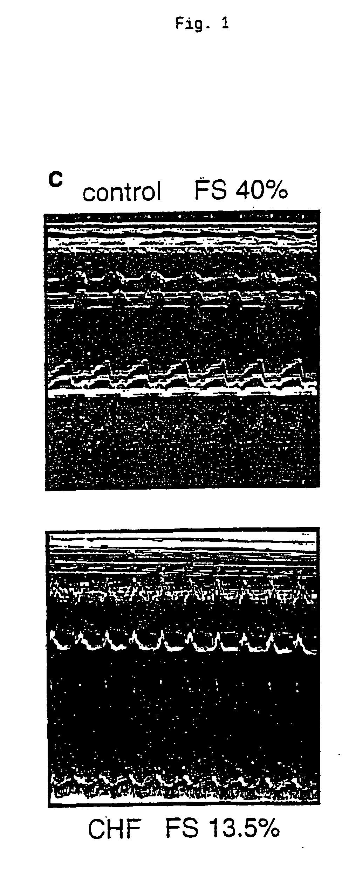

### Figur 1: Hämodynamische und Echokardiographische Charakterisierung des Herzinsuffizienz-Modells

Transthorakale "M-Modus"- echokardiographische Aufzeichnungen in Kontrolltieren (oben) und CHF-Tieren (unten). Die linksventrikulären Diameter sind durch einen Doppelpfeil angezeigt; EDD: enddiastolische Diameter; ESD: endsystolische Diameter. Nach zwei Wochen zeigen die Tiere mit Schrittmacher eine Kammererweiterung mit erniedrigten Wandbewegungen, was auf die herabgesetzte Herzfunktion und erhöhten Wandstress hindeutet.

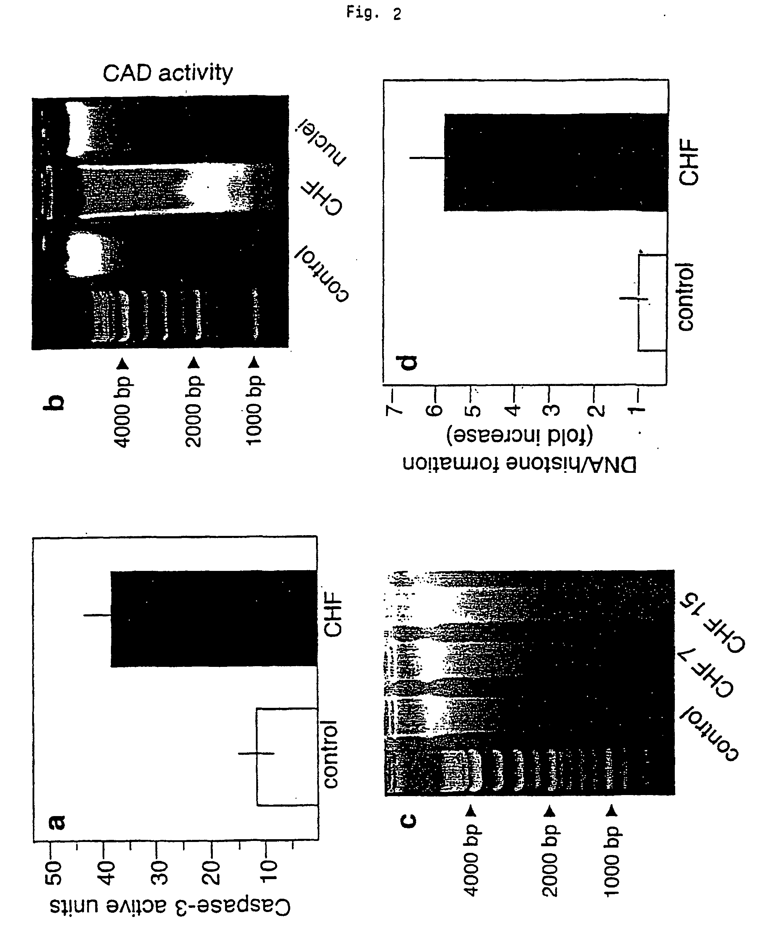

### Figur 2: Induktion der Caspase-3 vermittelten CAD-Aktivierung und anschließende DNA-Fragmentierung in isolierten ventrikulären Kardiomyocyten von Kontroll-Myokard und insuffizientem Myokard (CHF)

**a:** Fluorogener Assay der Caspase-3-Enzymspiegel gemessen in cytosolischen Fraktionen von isolierten Myocyten von Kontroll-Myokard und Myokard mit Schrittmacher (CHF); n=5 pro Gruppe, p < 0,005 ANOVA mit der Scheffé post-hoc-Analyse.
**b:** Fragmentierung genomischer DNA, die aus isolierten Kaninchenleber-Nuclei isoliert worden war und mit cytosolischen Extrakten von Kontroll-Myokard und insuffizienten Kardiomyocyten (CHF) inkubiert wurde. Die DNA-Fragmentierung spiegelt die Aktivität der Caspase-aktivierten Desoxyribonuclease (CAD) wider.
c: Isolierte DNA vom Herzen von insuffizienten Tieren nach 7 Tagen (CHF 7) und 15 Tagen (CHF 15) zeigte einen Anstieg der DNA-Leiterbildung ("laddering") im Vergleich zu Kontrollgewebe.
d: Freigesetzter DNA/Histon-Komplex wurde mit einem ELISA-System von Kontroll-Kardiomyocyten und Kardiomyocyten mit Schrittmacher quantifiziert.

Die Daten sind als Mittelwerte ± SEM ausgedrückt; n=5 pro Gruppe; p < 0,005.

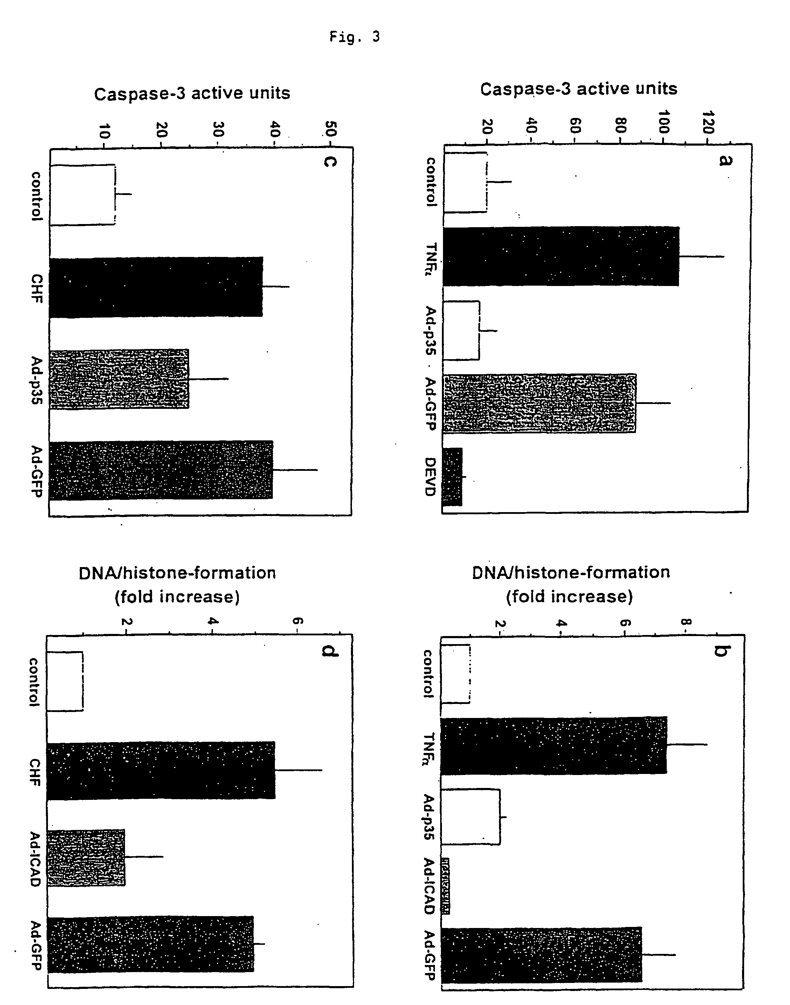

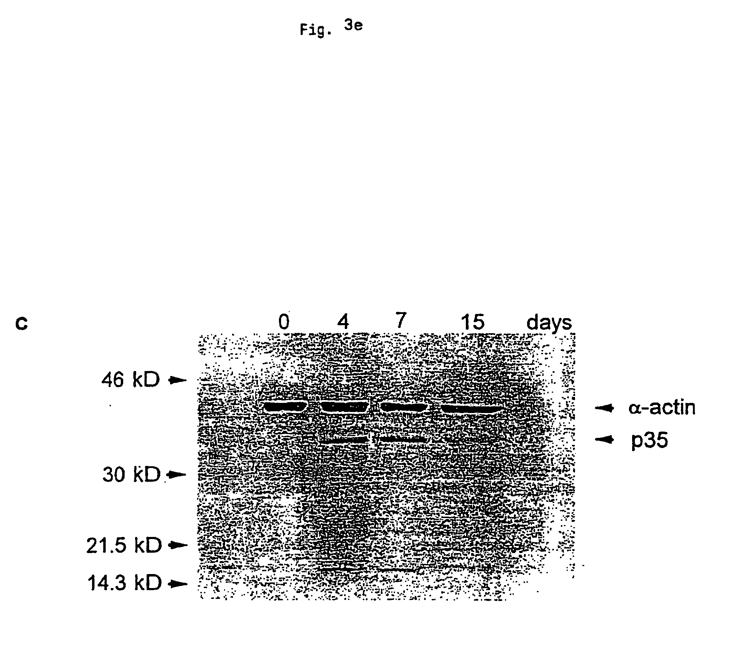

### Figur 3: Blockierung der Caspase-3-Aktivität und der DNA-Fragmentierung durch adenovirale Überexpression von p35 und ICAD in vitro (a,b) und in vivo (c,d)

**a:** Caspase-3-Aktivität wurde in isolierten Kontroll-Kardiomyocyten und TNFα-stimulierten Zellen in der Gegenwart eines Adenovirus-Konstrukts für p35 (Ad-p35), des leeren Konstrukts (Ad-GFP) und des Tetrapeptid-Inhibitors für Caspase-3 (DEVD) gemessen.
**b:** Die DNA/Histon-Bildung wurde in isolierten Kontrollzellen und TNFα-stimulierten ventrikulären Kardiomyocyten nach Adenovirus-Infektion mit Ad-p35, Ad-ICAD und Ad-GFP (als Kontrolle) bestimmt.
**c:** Kardiomyocyten wurden von Kontroll-Myokard und Myokard mit Schrittmacher (CHF) isoliert und die Caspase-3-Aktivität wurde in cytosolischen Extrakten gemessen. Die 3. und 4. Säule repräsentieren Enzymaktivitätsmesssungen von Kardiomyocyten vom Myokard der linken Kammer nach Adenovirus-Gentransfer von Ad-p35 und Ad-GFP (Kontrolle).
**d:** Ventrikuläre Myocyten wurden von Kontrollherzen und Herzen mit Schrittmacher (CHF) isoliert und die DNA/Histon-Bildung wurde in zellfreien Extrakten quantifiziert. Die 3. und 4. Säule repräsentieren die DNA-Fragmentierungsanalyse an Zellen von Tieren nach myokardialem Gentransfer mit Ad-ICAD und dem Kontroll-Adenovirus Ad-GFP.
***e:*** Immunblot-Analysen von zytosolischen Extrakten von der anterolateralen Wand von mit Ad-p35 infiziertem Myokard 0, 4, 7 und 15 Tage, nach Gentransfer. Die Immunfärbung wurde mit den beiden monoklonalen Antikörpern anti-Flag M2 und anti-α-Sarkomeraktin-Antikörper für p35 durchgeführt, um parallel die Expression von p35 und Aktin dokumentieren zu können..

Die Daten sind als Mittelwerte ± SEM ausgedrückt; n=4 pro Gruppe; p < 0,01, p < 0,001.

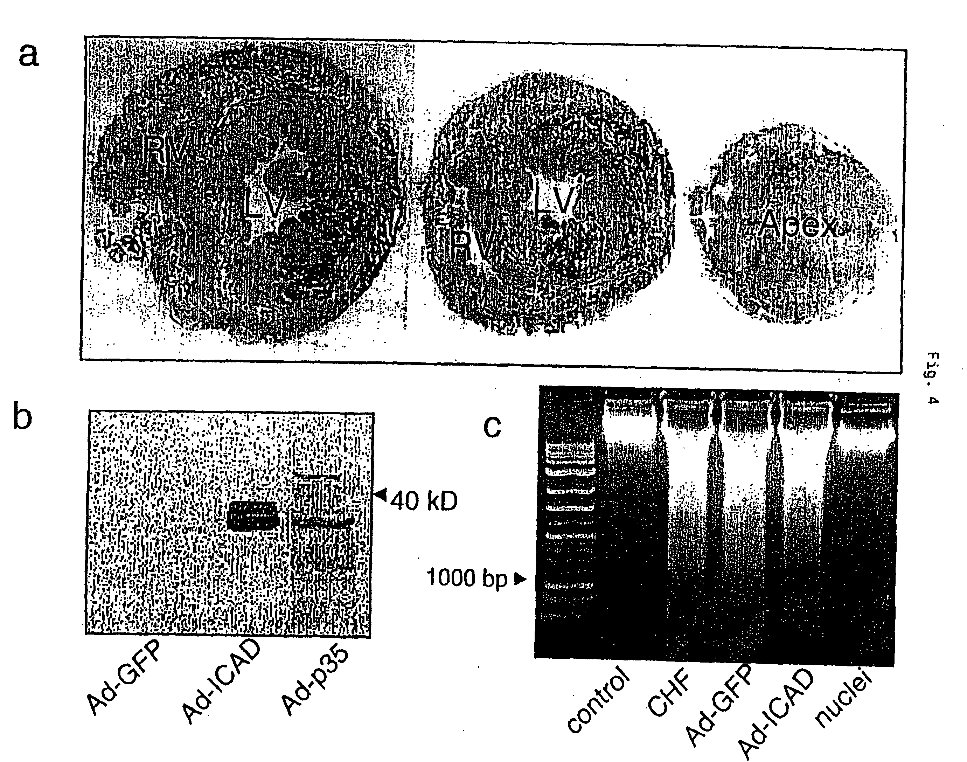

### Figur 4: Adenovirus-Gentransfer zum versagenden Myokard

**a:** Makroskopische Schnitte von Kaninchenherzen, bei denen ein β-Galactosidase codierendes Adenovirus Ultraschallgesteuert injiziert wurde (Schnittdicke: 7 µm, Abstand zwischen den einzelnen Schnitten: 200 µm). Die Schritte wurden mit X-Gal angefärbt. Das linksventrikuläre Myokard ist mit LV bezeichnet, das rechtsventrikuläre mit RV.
**b:** Immunblot-Anylase von cytosolischen Extrakten von mit Ad-GFP, Ad-ICAD und Ad-p35 infiziertem Myokard.
**c:** Fragmentierung genomischer DNA, die aus isolierten Kaninchenleber-Nuclei isoliert worden war, bei Inkubation mit cytosolischen Extrakten von isolierten ventrikulären Kontroll-Kardiomyocyten und insuffizienten Myocyten (CHF). Außerdem wurde die Aktivität der DNA-Fragmentierung von Kardiomyocyten nach Adenovirus-Gentransfer mit Ad-ICAD und Ad-GFP in vivo analysiert.

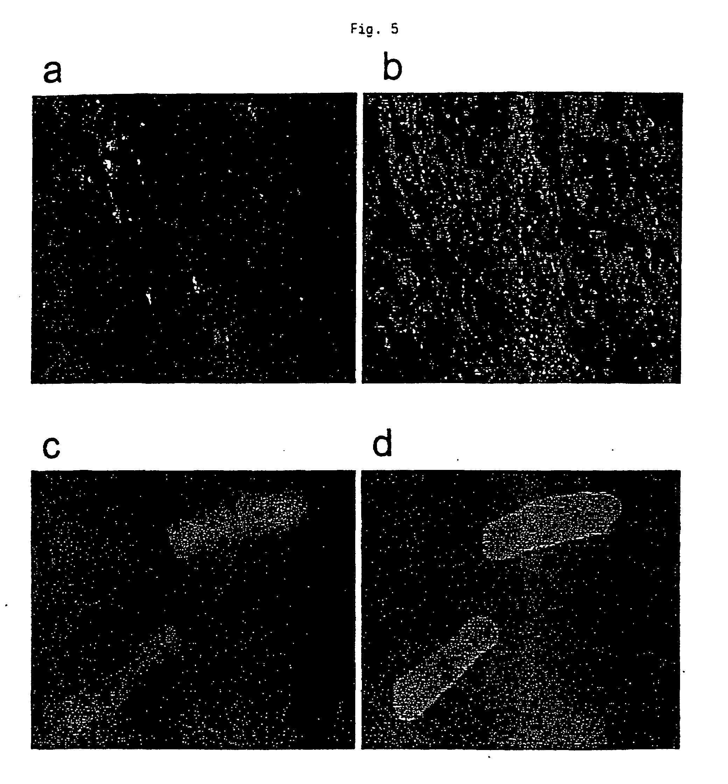

### Figur 5: Unter-Licht-Gewebeschnitte nach in vivo-Gentransfer mit den Adenovirus-Konstrukten für p35 und ICAD

**a:** Die GFP-Expression in makroskopischen Schnitten von infiziertem Myokard wurde durch Phasenkontrastfluoreszenzmikroskopie unter Verwendung eines 450-490 nm-Filters sichtbar gemacht.
**b**: Die direkte Transgen-Expression nach Ad-ICAD-Infektion wurde durch Immunfärbung mit einem monoclonalen anti-FLAG-Antikörper gegen das in beide Transgene eingeführte künstliche Epitop nachgewiesen. Die Probe wurde durch Fluoreszenzmikroskopie unter Verwendung eines 546 nm-Filters (Rhodamin-Fluoreszenz) sichtbar gemacht.
**c,d:** Ventrikuläre Kardiomyocyten wurden aus mit Ad-p35 infiziertem Myokard isoliert und die Transgen-Expression wurde durch Fluoreszenzmikroskopie für GFP und den anti-FLAG-Antikörper dokumentiert.

### Figur 6: Echokardiographische und hämodynamische Messungen an mit Ad-ICAD behandelten Herzen

Die Herzkatheterisierung wurde beim basalen Zustand und unter ansteigenden Adrenalin-Konzentrationen durchgeführt.
**a:** "fractional shortening" -Aufzeichnungen an Kontrollherzen und mit Ad-GFP oder Ad-ICAD infizierten Herzen nach 15 Tagen mit Schrittmacherversorgung. Der Prozentsatz an "fractional shortening" wurde als % FS=[(EDD-ESD)/EDD]x100 berechnet.
**b:** Die linksventrikulären enddiastolischen Diameter sind für die Kontrolle und für mit Ad-GFP oder Ad-ICAD infiziertem Myokard nach einem Schrittmacherzeitraum von 15 Tagen gezeigt.
**c:** Die linken ventrikulären enddiastolischen Drücke wurden für die Kontrolle und für Ad-GFP- oder Ad-ICAP-Tiere nach der Schrittmacherversorgung bestimmt.
**d:** Die Entwicklung der LVEDP während des Zeitraums der Schrittmacherversorgung ist für Ad-GFP-, Ad-ICAD- und Kontrolltiere nach 7 bzw. 15 Tagen dargestellt.
**e:** dp/dtmax-Aufzeichnungen von Kontrolltieren und mit Ad-ICAD behandelten Tieren nach Verabreichung von ansteigenden Konzentrationen von Adrenalin. Die Daten sind als Mittelwerte ± SEM dargestellt.

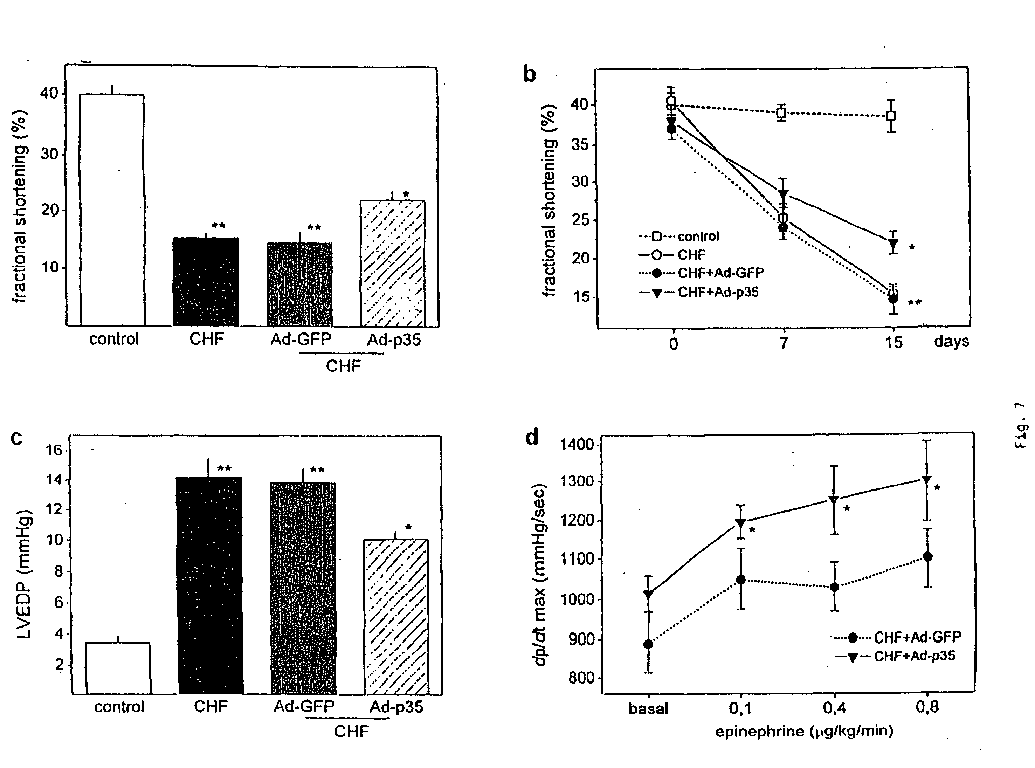

### Figur 7: Echokardiographische und hämodynamische Messungen an mit Ad-p35 behandelten Herzen

Die Infektion mit den Adenovirus-Konstrukten wurde vor dem Start der Schrittmacherperiode durchgeführt.
**a:** "fractional shortening" wurde in der Kontrolle, CHF und 15 Tage nach der transkoronären Verabreichung von Ad-GFP oder Ad-p35 an mit Schrittmacher versorgte Herzen abgeschätzt.
**b:** Zeitverlauf des "fractional shortening" nach 7 und 15 Tagen Schrittmacherversorgung.
**c:** Der verringerte enddiastolische Kammerdruck (LVEDP) wurde in der Kontrolle, CHF und 15 Tage nach der transkoronären Verabreichung von Ad-GFP oder Ad-p35 an mit Schrittmacher versorgten Herzen abgeschätzt.
**d:** dp/dtmax-Aufzeichnungen nach Verabreichung von ansteigenden Dosen an Epinephrin. Die Daten sind als Mittelwerte ± SEM (n=8 pro Gruppe; * p<0,05; ** p<0,001) im Vergleich zur Kontrolle und CHF und Ad-GFP-Herzen dargestellt.

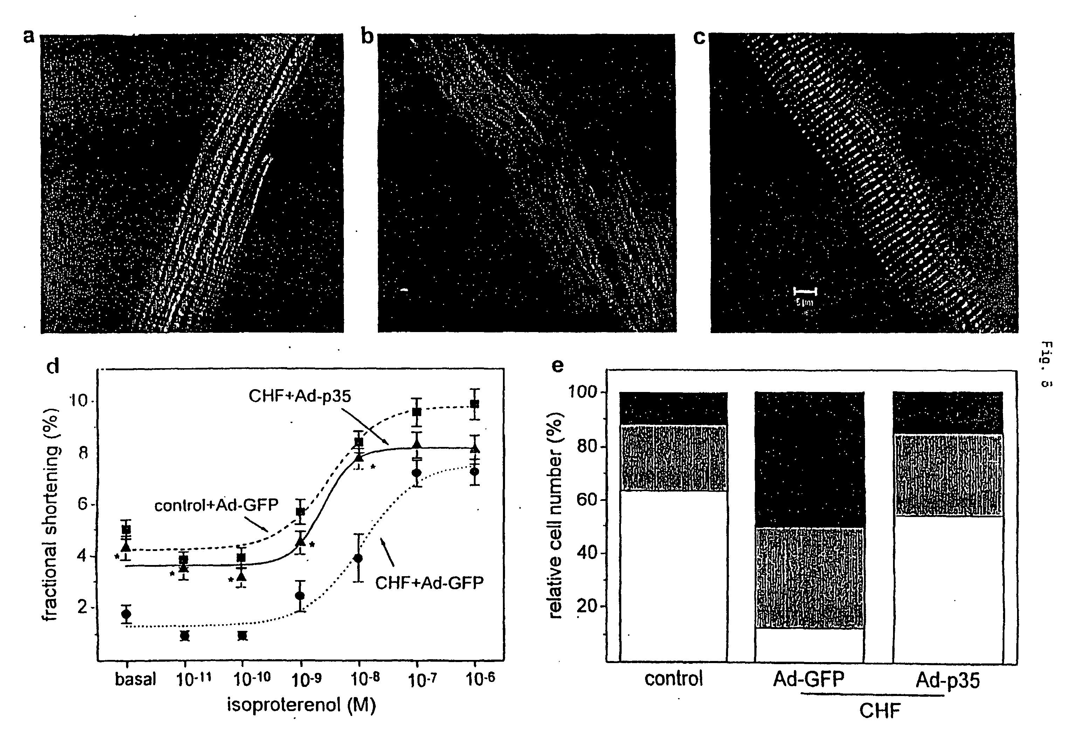

### Figur 8: Auswirkung der p35-Expression auf die Sarkomerorganisation und die Kontraktionskraft von Muskelzellen von mit Schrittmacher versorgten Herzen

**a,b,c:**Ventrikuläre Kaninchen-Herzmuskelzellen wurden von der anterolateralen Wand der Kontrolle (a), CHF (b) und Ad-p35-infiziertem insuffizientem Myokard (c) isoliert und nach Phalloidin-Anfärbung über Konfokale Laser-Scanning-Mikroskopie sichtbar gemacht.
**d:** Kontraktionsamplitude in isolierten Herzmuskelzellen (n=60 Zellen von vier Kaninchen pro Gruppe).
**e:** Die Morphologie von Phalloidin-gefärbten Muskelfasern wurde semiquantitativ auf der Basis der von organisiertem Sarkomer in dem Gesamtzellbereich besetzten Gebiet bewertet: schwach, weniger als 1/3 des Zellbereichs (schwarzer Bereich); mäßig, weniger als 2/3 des Zellbereichs (grauer Bereich); gut, Gesamtzellbereich (leerer Bereich). Für jede Gruppe wurden 120, von 4 Tieren isolierte Zellen gezählt. Daten (in d) wurden als Mittelwert ± SEM ausgedrückt (* p<0,001 im Vergleich zu mit Schrittmacher versorgtem Myokard (CHF+Ad-GFP)).

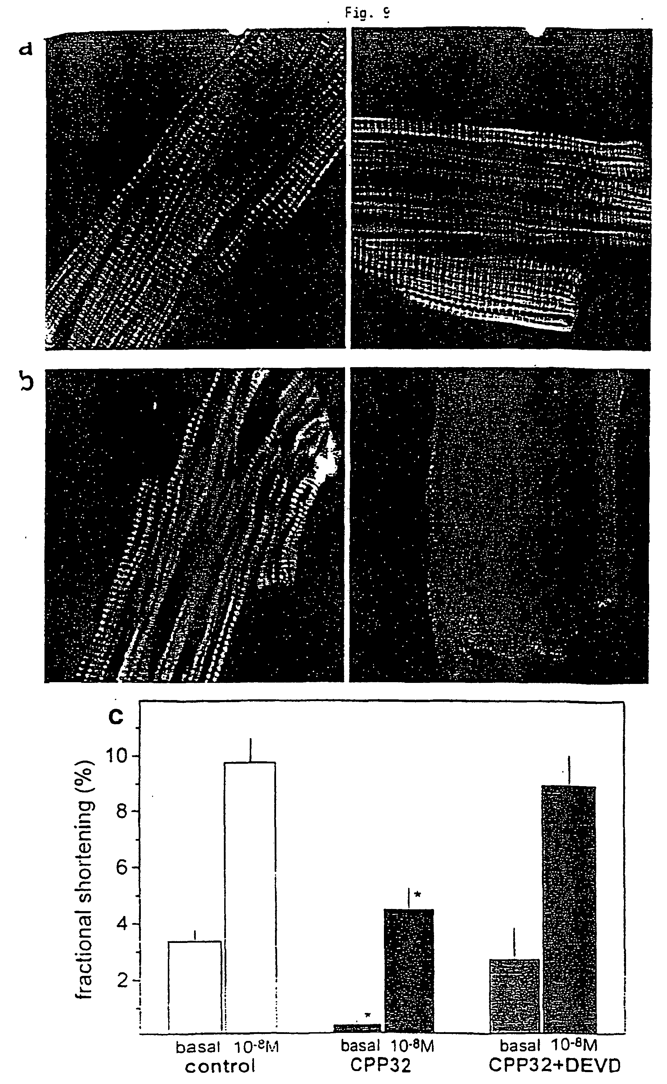

### Figur 9: Mikroinjektion von aktivierter Caspase-3 in das Zytoplasma von gesunden ventrikulären Herzmuskelzellen

**a,b:** FITC-konjugiertes Dextran allein (a) oder FITC-konjugiertes Dextran + humane rekombinante aktive Caspase-3 (b) (linke Abbildung: 4 ng/µM; rechte Abbildung: 20 ng/*µL) wurde in Muskelzellen injiziert. Die Morphologie der Aktinfasern wurde mittels Konfokale Laser-Scanning-Mikroskopie nach Phalloidin-Anfärbung sichtbar gemacht.*
**c:** Die Kontraktionsamplitude unter Basalbedingungen und Isoprenalin-Stimulation (10⁻⁸ mol/L) wurde in den Zellen nach Mikroinjektion von FITC-konjugiertem Dextran (als Kontrolle) oder Caspase-3 (CPP32) bestimmt. Es wurde auch eine Vorinkubation mit DEVD-fmk (R and D Systems, Wiesbaden, Deutschland) durchgeführt (graue Balken). n=45 Zellen für jede Gruppe. Die Daten in c sind als Mittelwert ± SEM gezeigt (* p<0,005 im Vergleich zu injizierten Kontrollzellen (basal; 10⁻⁸ mol/*L Isoprenalin)*.

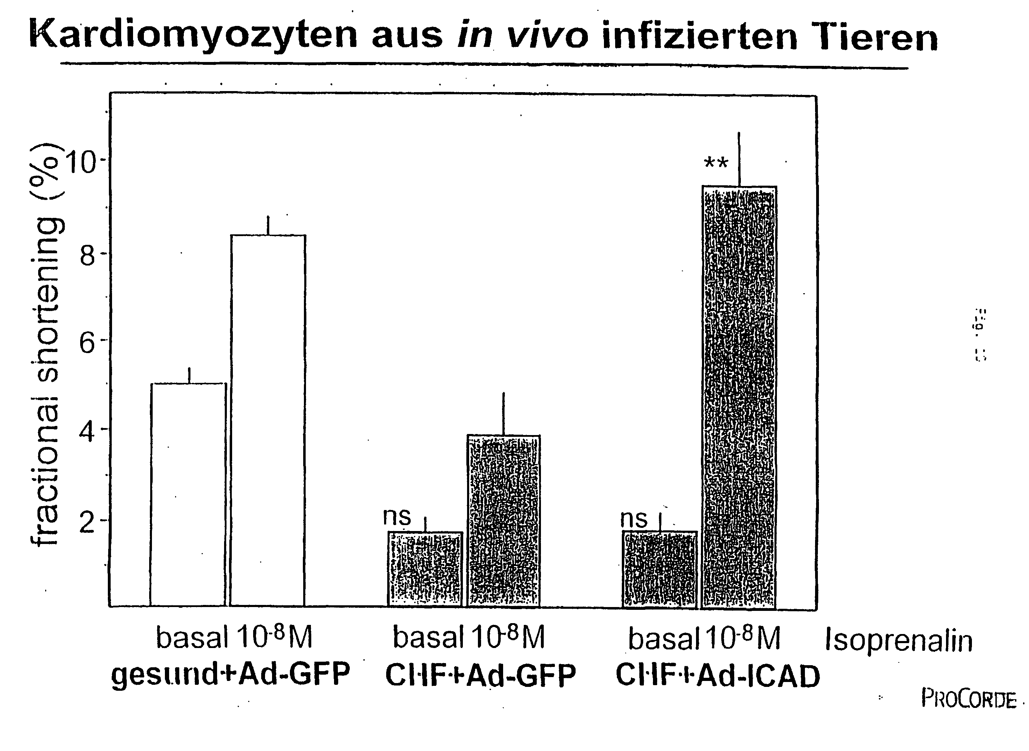

### Figur 10: Auswirkung von ICAD auf die Kontraktilität einzelner Herzmuskelzellen

Kontraktionsamplitude in isolierten Herzmuskelzellen (n=60 Zellen von vier Kaninchen pro Gruppe). Versuch wurde wie bei Fig. 8d beschrieben durchgeführt; n.s. = nicht signifikant, ** = p < 0,05.

Die folgenden Beispiele erläutern die Erfindung.

### Beispiel 1:

### Allgemeine Verfahren

### (A) Konstruktion und Reinigung von rekombinantem Adenovirus

Rekombinanten, den Inhibitor der Caspase-aktivierten DNAse (ICAD) der Ratte oder den Baculovirus Apoptose-Suppressor p35 codierende Adenoviren (El- und E3 defizient; Serotyp 5) wurden wie von He et al., Proc. Natl. Acad. Sci. USA, 95, (1988), 2509-2514 beschrieben hergestellt. Dazu wurde die ICAD oder p35 codierende, mit dem am N-Terminus "Flag"-Epitop (Stratagene, La Jolla, CA, USA) versehene Sequenz in die Polylinkersequenz des GFP exprimierenden "pAdTrack"-Vektors zwischen einen Gewebe-unspezifischen Cytomegalivirus(CMV)-Promotor und ein SV40-Polyadenylierungssignal inseriert (Enari et al., Nature 391, (1988), 43-50), (Davidson and Steller, Nature 391, (1998), 587-591), (He et al., supra), (Inan et al., Onkogene 13, (1996) 749-755). Das erhaltene Plasmid wurde dann zusammen mit dem "pAdEasy-1"-Plasmid in elektrokompetente BJ5138 Bakterien (He et al., supra) cotransformiert. Zur Herstellung eines nur das Gen für verstärktes GFP enthaltenden Kontrolladenovirus wurde eine Cotransformation mit den Plasmiden "pAdTrack" und "pADEasy" durchgeführt. Rekombinante Adenovirusvektor-DNA wurde aus positiven Clonen extrahiert und unter Verwendung eines "SuperFectTM"-Transfektionsreagenz (QIAGEN, Hilden, Deutschland) in subconfluente HEK 293 Zellen transfiziert. Aus dem Zellysat wurden rekombinante Viren (Ad-ICAD, Ad-p35 und Ad-GFP) erhalten und diese wurden mittels PCR und Restriktionsspaltungen analysiert.

Nach der Isolierung wurden durch Infektion von subconfluenten HEK 293-Zellen in 150-mm Platten (moi: 5 pfu/Zelle) rekombinante Adenoviren in großem Maßstab präpariert. 48 bis 72 Stunden nach Infektion nach Eintreten der cytopathogenen Wirkung wurden die Zellen durch Abkratzen und eine 20minütige Zentrifugation bei 1.000 x g geerntet. Das Zellpellet wurde in PBS und 0,25% Triton-X 100™ resuspendiert. Das Homogenat wurde 10 Minuten bei Raumtemperatur inkubiert und die Nuclei der geöffneten HEK-Zellen durch einen zentrifugationsschritt bei 2.500 x g (20 Minuten) entfernt. Der Überstand mit den Viruspartikeln wurde auf einen CsCl-Stufengradienten gegeben (1,3 und 1,4 g CsCl/ml, gelöst in TE-Puffer) und 1,5 Stunden bei 10°C mit 150.000 x g ultrazentrifugiert. Die sich in der 1,3-1,4 g/ml-Zwischenphase bildende Virusbande wurde mit einer Spritze entfernt, gegen 1% Saccharose und 10% Glycerin enthaltende PBS dialysiert und in Aliquots bei -80°C aufbewahrt. Adenovirus-Titer wurden mittels Plaquetitration auf HEK 293-Zellen (Krown et al., J.Clin.Invest.98 (1996), 2854-2865) bestimmt.

### (B) Zellkultivierung und Adenovirus-Infektion von H9c2-Kardiomyoblasten

H9c2-Kardiomyoblasten (ATCC CRL 1446, Ratten-Kardiomyoblasten) wurden in Monolayern in DMEM, 1.0% FBS, 2 mmol/l Glutamin, Penicillin (100 IE/ml) und Streptomycin (100 µg/ml) in 10% CO₂ bei 37°C in einem befeuchteten Inkubator gezüchtet. Nachdem die Kardiomyoblasten 70 bis 80% Konfluenz erreicht hatten wurden sie mit einem geeigneten Adenovirus-Titer in PBS transfiziert. Nach einer Stunde Inkubation bei Raumtemperatur wurde das Kulturmedium zu den Platten zurückgegeben. 36 bis 48 Stunden nach Adenovirus-Infektion wurden die Zellen für die einzelnen Experimente verwendet.

### (C) Präparation und Züchtung von adulten ventrikuären Kardiomyocyten von Ratten und Kaninchen

Einzelne Calcium-tolerante ventrikuläre Myocyten wurden aus 12-16 Wochen alten Wistar-Ratten (300-450 g) oder aus 3 Monate alten männlichen Neuseeland-Kaninchen (2,8-3 kg) isoliert. Die exzidierten Herzen wurden über die Aorta mit Ca²⁺-freiem "Powell"-Medium (110 mmol/l NaCl, 2,5 mmol/l KCl, 1,2 mmol/l KH₂PO₄, 1,2 mmol/l MgSO₄, 11 mmol/l Glucose, 25 mmol/l NaHCO₃, äquilibriert mit 5% CO₂ und auf einen pH-Wert von 7,4 eingestellt) bei konstanter Durchflußgeschwindigkeit perfundiert. Nach 10 Minuten wurde ein enzymatischer Verdau durch Austausch des Ca²⁺-freien Puffers gegen 110 bzw. 218 IE/ml Kollagenase Typ II (Worthington, Freehold, New Jersey, USA) und 30 bzw. 40 µmol/l Ca²⁺ (für Ratte bzw. Kaninchen) enthaltendes "Powell"-Medium ausgetauscht. Nach gutem Verdau des Gewebes wurde das Herz von der Perfusionsvorrichtung entfernt, der Herzkammermuskel freigelegt und mechanisch in "Powell"-Medium unter ständiger Oxygenierung dissoziiert. Nach Filtration über eine Nylonmmebran (200 µm Maschenweite) wurde die Zellsuspension 3 Minuten bei 20 x g zentrifugiert und die Zellen wurden in 0,2 mmol/l Ca²⁺ enthaltendem "Pöwell"-Medium resuspendiert. Die Zellen wurden sich absetzen gelassen, das Pellet in 0,4 mmol/l Ca²⁺ enthaltendem "Powell"-Medium gesammelt und vorsichtig auf einen 4% Rinderserumalbumin(BSA)-Gradienten in "Powell"-Medium (1 mmol/l Ca²⁺) gegeben. Nach 2 Minuten Zentrifugation bei 20 x g wurden die Kardiomyocyten in M199 Kulturmedium (supplementiert mit MEM-Vitaminen, MEM nicht-essentiellen Aminosäuren, 25 mmol/l HEPES, 10 µg/l Insulin, 100 IE/ml Penicillin, 100 µg/ml Streptomycin und 100 µg/ml Gentamicin) resuspendiert, auf Laminin-vorbeschichteten Schalen (5-10 µg/cm²) bei einer Dichte von 10⁵ Zellen pro cm² ausplattiert und in einer befeuchteten Atmosphäre (5% CO²) bei 37°C gezüchtet. Die Infektion der Kardiomyocyten mit den Adenoviren erfolgte 6 bis 8 Stunden nach dem Ausplattieren in M199-Kulturmedium. Nach Isolierung der Zellen von normal oder ungenügend in vivo-infizierten Kaninchen und Verwendung zur Bestimmung des Auftretens von Apoptose wurden sie unmittelbar nach Zentrifugation im 4% BSA-Gradienten direkt als Pellet bei -80°C eingefroren.

### (D) Westernblot-Analyse

(I) Zum immunologischen Nachweis der induzierten Proteine GFP, ICAD und p35 wurden die H9c2-Cardiomyoblasten 48 Stunden nach der Infektion (moi: 50 pfu/Zelle) in 10 mM HEPES-Puffer, pH-Wert 7,0 (der 40 mM β-Glycerinphosphat, 50 mM NaCl, 2 mM MgCl₂, 5 mM EGTA, 1 mM DTT, 2 mM ATP, 10 mM Creatinphosphat, 50 µg/ml Creatinkinase, 1 mM PMSF, 1 µg/ml Leupeptin, 1 µg/ml Pepstatin, 10 µg/ml Aprotinin, 16 µg/ml Benzamidin, 10 µg/ml Phenantrolin enthielt) geerntet und durch vier Gefrier/Auftau-Zyklen aufgebrochen. Die erhaltenen Lysate wurden 30 Minuten bei 15.000 UpM zentrifugiert und die Proteinkonzentrationen in einem "Bradford"-Assay bestimmt. Gleiche Proteinmengen (50-200 µg) wurden mit SDS-Auftragspuffer verdünnt, auf einem 12% . Polyacrylamidgel aufgetrennt und elektrophoretisch auf eine Nitrozellulose-Membran (Bio-Rad Laboratories, München, Deutschland) übertragen. Die Blots wurden zur Überprüfung des Proteintransfers mit "Ponceau"-Rot angefärbt. Nach Blockierung über Nacht mit 5% fettfreiem Milchpulver in TBST (10 mM Tris-HCl, pH-Wert 8,0, 150 mM NaCl, 0,05 Tween-20™) wurden die Membranen 1 Stunde mit anti-FLAG M2-Antikörper (Stratagene) inkubiert, der 1:10.000 (ICAD-Nachweis) oder 1:1.000 (p35-Nachweis) in TBST (mit 0,1% BSA und 0,02% Na-Azid) verdünnt war. Antigen/Antikörper-Komplexe wurden nach einstündiger Inkubation der Membranen mit 1:10.000 verdünntem, mit Meerrettichperoxidase konjugiertem anti-Maus-IgG (Sigma-Aldrich Chemie GmbH, München, Deutschland) über Chemilumineszenz sichtbar gemacht ("ECL"-Nachweiskit, Amersham Pharmacia Biotech, Wien, Österreich).
(II)Native ICAD-Spaltprodukte in Lysaten von scheinoperiertem und versagendem Myokard wurden unter Verwendung von polyklonalen Ziegren-Antikörpern gegen den N-Terminus von Maus-ICAD (St. Cruz Biotechnology, Santa Cruz, U.S.A.) (Verdünnung: 1:1.000) nachgewiesen. Einige Extrakte von Kontrollherzen wurden auch 30 Min. bei 37°C mit 1 ng/µL rekombinanter humaner aktiver Caspase-3 in Gegenwart oder Abwesenhei t des Tetrapeptid-Caspase-3-Inhibitors DEVD-fmk (R and D Systems, Wiesbaden, Deutschland) (100 µmol/L) inkubiert. Der immunologische Nachweis von p35 und α-Sarkomer-Aktin in Extrakten von transkoronarem scheinoperiertem Myokard wurde mittels des monoklonalen Maus-anti-FLAG M2-Antikörpers (Verdünnung: 1:1000) und eines monoklonalen anti-α-Sarkomer-Aktin-Antikörpers (Sigma, Taufkirchen, Deutschland) (Verdünnung: 1:5000) durchgeführt.

### (E) Immuncytochemische und mikroskopische Untersuchungen

Isolierte adulte ventrikuläre Kardiomyocaten, die auf mit 5 µg/cm² Laminin beschichteten Mikroskop-Deckgläschen ausplattiert worden waren, wurden mit Kontroll-Adenovirus Ad-GFP oder mit Ad-ICAD- bzw. Ad-p35-Viren (moi: 50 pfu/Zelle) infiziert und 48 Stunden nach Infektion analysiert. Zur Bestimmung der Expression der Transgene in in vivo infizierten Kaninchenherzen wurde gefrorenes Gewebe auf einem Gefriermikrotom in Scheiben mit einer Dicke von 3 bis 4 µm geschnitten. Nach dreimaligem Spülen mit PBS wurden die Zellen und Schnitte 15 Minuten mit 4% Paraförmaldehyd in PBS fixiert und dann 10 bzw. 30 Minuten mit 0,1% Saponin in PBS permeabilisiert. Zur Vermeidung einer unspezifischen Antikörper-Bindung wurden die Kardiomyocyten vor der Markierung mit dem monoclonalen Maus-anti-FLAG M2 (IgG1)-Antikörper (Stratagene, 10 µg/ml) 30 Minuten mit 10% FBS in DMEM behandelt. Nach Inkubation mit einem Rhodamin-konjugiertern anti-Maus-IgG (Santa Cruz Biotechnology, Santa Cruz, CA, USA; 4 µg/ml) wurden die Proben mittels Phasenkontrast-Fluoreszenzmikroskopie unter Verwendung eines 450-490 nm-Filters (GFP-Fluoreszenz) und eines 546 nm-Filters (Rhodamin-Fluoreszenz) sichtbar gemacht (inverses Mikroskop "Axiovert 25", Zeiss, Jena, Deutschland).

### (F) ELISA für Histon-gebundene DNA-Fragmente

Apoptose in adulten ventrikulären Kardiomyocyten wurde mit einem im Handel erhältlichen quantitativen, auf einem gegen DNA und Histone gerichteten monoclonalen Maus-Antikörper verwendenden Nucleosom-ELISA (Roche Diagnostics, Mannheim, Deutschland) bestimmt. Die Menge an Nucleosomen in dem Lysat von 2 x 10³ Zellen wurde über die in dem Immunkomplex zurückbehaltene Peroxidase bestimmt und photometrisch ausgewertet. Jeweils drei Proben wurden ausgewertet, wobei die optische Dichte (OD) bei 405 nm gemessen wurde. Der die Apoptose erhöhende Faktor wurde für jede Experimentgruppe als OD (Behandlung) / OD (Kontrolle) nach Abzug der Hintergrund-OD₄₀₅ berechnet.

### (G) Caspase-3-Aktivität

Die Aktivität von Caspase-3 wurde mit dem colorimetrischen "CPP32"-Assaykit (Clontech Laboratories GmbH, Heidelberg, Deutschland) durch den Nachweis von Chromophor-p-Nitroanilid nach Spaltung von dem markierten Substrat Asp-Glu-Val-Asp(DEVD)-p-nitroanilid bestimmt. Dazu wurden 2 x 10⁶ adulte Kardiomyocyten lysiert und gleiche Proteinmengen mit 50 µmol/l DEVD-p-Nitroanilid 1 Stunde bei 37°C zur Reaktion gebracht. Die Aktivität wurde photometrisch bei 405 nm bestimmt und die Ergebnisse wurden mit bekannten Konzentrationen von p-Nitroanilid kalibriert. Die Einheiten an Protease-Aktivität wurden als die Menge an Caspase-3 definiert, die zur Erzeugung von 1 pmol p-Nitroanilid bei 25°C erforderlich ist.

### (H) ICAD-Aktivitätsassay

ICAD-Aktivität wurde durch Bestimmung der Hemmung von CAD in der Fragmentierung von DNA aus Kaninchenleber-Nuclei ermittelt. Die Nuclei wurden wie von Blobel und Potter (Science 154 (1966), 1662) beschrieben präpariert und bei -80°C aufbewahrt.

H9c2 Kardiomyoblasten wurden mit Ad-GFP oder Ad-ICAD (moi: 50 pfu/Zelle) infiziert und 48 Stunden später wurde die CAD-Aktivität durch Behandlung der Zellen mit Ratten-α-TNF (500 E/ml) für 5 Stunden stimuliert. 100 µg Protein aus Rohzellysaten wurden mit 2 x 10⁶ Nuclei in einem Reaktionspuffer inkubiert, der aus 10 mM HEPES, pH-Wert 7,0, 40 mM β-Glycerinphosphat, 50 mM NaCl, 2 mM MgCl₂, 5 mM EGTA, 1 mM DTT, 2 mM ATP, 10 mM Creatinphosphat, 50 µg/ml Creatinkinase und einem Gemisch von Proteaseinhibitoren ( 1 mM Phenylmethylsulfonylfluorid (PMSF), 10 µg/ml Leupeptin, 1 µg/ml Pepstatin, 10 µg/ml Aprotinin, 16 µg/ml Benamidin, 10 µg/ml Phenantrolin) bestand. Nach 90 Minuten Inkubation bei 37°C wurden die Nuclei durch Zentrifugation beio 35.000 x g (5 Minuten) gewonnen und danach 30 Minuten bei 56°C in 100 mM Tris-HCl, pH-Wert 8,5, 5 mM EDTA, 0,2 M NaCl, 0,2% SDS, 1 mg/ml Proteinase K lysiert. Danach wurde die DNA durch Zugabe des gleichen Volumens Isopropanol präzipitiert, in 20 µl Tris-HCl, pH-Wert 8,5 (mit 1 mM EDTA und 1 mg/ml RNase A) gelöst und 30 Minuten bei 37°C inkubiert. Die DNA wurde durch Gelelektrophorese (1% Agarose in Gegenwart von 0,5 µg/ml Ethidiumbromid) analysiert.

### (I) Gentransfer und Eingriffe am Tier

Adulte männliche weisse Neuseeland-Kaninchen (2-4 kg) wurden mit Midazolam (2 mg/kg) und Medetomidin (150 µg/kg) anästhesiert, intubiert und künstlich beatmet. Die linke Brustwand wurde eröffnet und zwei isolierte Schrittmacherkabel wurden am linken Herzen angebracht. Nach diesem Vorgehen wurden die Tiere extubiert. Bei den Kontrolltieren wurde ein Brustwandschnitt mit Implantierung der Schrittmacherkabel durchgeführt, es wurde jedoch niemals der Schrittmacher in .Gang gesetzt. Zwölf Stunden nach diesem Eingriff wurde eine Schrittmachergeschwindigkeit von 360 Schlägen/Minute eingeleitet. Dies wurde zu Beginn der Untersuchungen und dann wöchentlich ( insgesamt über einen Zeitraum von 15 Tagen) mittels eines EKG überprüft. Der Adenovirus-Gentransfer in den Kaninchen-Herzmuskel wurde nach bekannten Protokollen durchgeführt (Weig et al., Circulation 101, (2000), 1578-1585).

### (J) Haemodynamische und echokardiographische in vivo-Daten

Die Kontraktionskraft der linken Herzkammer wurde vor und 7 bzw. 15 Tage nach dem Adenovirus-Gentransfer untersucht. Die Kaninchen wurden wie vorstehend beschrieben anästhesiert. Die echocardiographischen Aufzeichnungen (M-Modus) wurden wie in früheren Studien beschrieben durchgeführt (Gardin et al., Circ. Res. 76 (1995), 907-914). Zusätzlich wurden EKG und Blutdruck kontinuierlich überwacht. Nach Präparation der rechten Karotis wurde ein mit einer Differenzierungsvorrichtung verbundener "Millar 2.5 French tip"-Katheter (Hugo Sachs, Freiburg, Deutschland) in die linke Herzkammer geführt. Die Überprüfung der Position des Katheters erfolgte sowohl mittels Fluoroskopie als auch durch Beobachtung der Blutdruckwellenform. Nach der Festlegung der basalen Kontraktionskraft und des Drucks der linken Herzkammer (mit ausgeschaltetem Schrittmacher) wurden 200 µl 0,9% NaCl als negative Kontrolle injiziert. Nach einer ausreichenden Äquilibrationsdauer wurde Adrenalin in Konzentrationen von 0,1 bis 0,8 ng injiziert. Messungen wurden vor den chirurgischen Eingriffen und 7 bzw. 15 Tage nach dem Schrittmacherzeitraum durchgeführt.

### (K) Statistische Analysen

Die Daten stellen die Mittelwerte ± SEM von mehr als drei unabhängigen Experimenten dar. Die Daten wurden mittels der "one-way" Varianzanalyse (ANOVA) und im Anschluß daran mittels der "Scheffé"-post-hoc-Analyse auf statistisch relevante Unterschiede geprüft.

### (L) Mikroinjektion

Die Mikroinjektionsexperimente wurden an frisch isolierten Herzmuskelzellen von scheinoperierten Kaninchen mittels eines "Femto-Jet"-Mikroinjektionsgeräts (Eppendorf, Hamburg, Deutschland) durchgeführt. FITC-konjugiertes Dextran (6 mg/ml) allein oder in Kombination mit humaner rekombinanter aktiver Caspase-3 (4 ng/µL und 20 ng/µL) in 5 mmol/L Kaliumphosphatpuffer (pH-Wert 7,4; 100 mmol/L KCl) wurden in das Zytoplasma der Zellen in Kulturmedium injiziert (Pᵢ = 1000 hPa, tᵢ = 0,1 Sek., P_{c} = 30 hPa), das mit 200 µmol/L BDM (Butanedionemonoxin, Sigma, Taufkirchen, Deutschland), 10 µmol/L Verapamil und optional 100 µmol/L DEFD-fmk supplementiert worden war. Nach zweistündiger Inkubation wurden Kontraktionsmessungen oder eine Phalloidin-Färbung durchgeführt. Injizierte Zellen wurden über FITC-Fluoreszenz selektiert.

### Beispiel 2:

### Physiologische Auswirkungen von chronischer Tachykardie in Kaninchen

Es wurde ein Model der Stauungsinsuffizienz des Herzens (CHF) mit geringem Minutenvolumen verwendet, das Veränderungen beim Menschen auf der haemodynamischen und biochemischen Ebene entspricht. In dieser Untersuchung starb keines der Tiere während der chirurgischen Instrumentierung, allerdings ergab sich eine Sterblichkeitsrate von 10-20% während des Zeitraums von 15 Tagen, in denen der Schrittmacher angeschlossen war. 15 Tage chronische Schrittmacherversorgung bei 360 Schlägen pro Minute führte zu dem allgemeinen klinischen Befund der systemischen Herzinsuffizienz einschließlich einer Erweiterung beider Kammern (Figur 1c), Pleuraergüssen und abdominalem Ascites. Echokardiographische Untersuchungen ergaben einen Anstieg des Ausmaßes der Enddiastole der linken Kammer und eine Erniedrigung des "fractional shortening" in den CHF-Kaninchen (Tabelle 1 (a)). Haemodynamische Messungen ergaben ebenfalls einen höheren enddiastolischen Druck der linken Kammer und eine geringere Kontraktionskraft der linken Kammer (bestimmt durch LV +dP/dt) und Relaxation (bestimmt durch LV-dP/dt) in den mit Schrittmacher versorgten Kaninchen (Tabelle 1 (b)). Die echokardiographischen und hämodynamischen Messungen wurden bei jedem Käninchen vor der Implantation des Schrittmachers und dann erneut nach 7 bzw. 15 Tagen (mit ausgeschaltetem Schrittmacher) aufgezeichnet. Somit wurde jedes Kaninchen als seine eigene Kontrolle verwendet. Scheinoperierte Kaninchen zeigten keinen Unterschied hinsichtlich hämodynamischer Parameter. In dem Myokard mit Insuffizienz wurden biochemische Veränderungen der β-adrenergen Signaltransduktion ähnlich denen bei der Herzinsuffizienz beim Menschen beobachtet. Die Dichte an β-adrenergem Rezeptor war in den insuffizienten Myocyten signifikant verringert und die Expression von βARK1 war erhöht.

Zur Kontrolle wurden Werte vor der Schrittmacher-Implantation und CHF-Werte drei Wochen nach Schrittmacher-Implantation bestimmt. HR, Herzrate [Schläge/min (bpm)]; LVEDD, LV end-diastolischer Diameter; LVESD, LV end-systolischer Diameter; FS, "fractional shortening", bestimmt als % FS = [(EDD-ESD)/EDD] x 100; LVEDP, LV end-diastolischer Druck; LVSP, LV end-systolischer Druck; dp/dtmax, Maximalrate der LV Druck-Zunahme; dp/dtmin, Maximalrate der LV Druck-Abnahme.

### Beispiel 3:

### Apoptose-Parameter bei Herzinsuffizienz

Um die wichtigsten biochemischen Merkmale der Apoptose in den Herzen der mit Schrittmacher versehenen Kaninchen beurteilen zu können, wurden adulte ventrikuläre Myocyten isoliert und eine Reihe von molekularen Analysen durchgeführt. Im Endstadium des Herzversagens wird die Apoptose im intakten Myokard von einem DNA-Abbau begleitet. Zur Untersuchung der Fähigkeit der Zellysate von insuffizienten Kardiomyocyten zur Bewirkung des DNA-Abbaus in vitro wurden Lebernuclei mit Lysaten von versagenden Myocyten inkubiert und die DNA wurde mittel Agarosegel-Elektrophorese untersucht (Fig.2a). Insuffizientes Myokard zeigte im Vergleich zu Kontrollgewebe eine erhöhte Aktivität für DNA-Abbau. Diese Aktivität konnte durch Überexpression von ICAD blockiert werden. Zusätzlich ergab sich in Gesamtzellextrakten, die von insuffizienten und Kontroll-Kardiomyocyten präpariert worden waren, eine signifikante Erhöhung ( etwa 3 fach) der Caspase-3-Aktivität in den CHF-Zellen (Fig.2b). Cytosol-Extrakte vom Myokard von mit Schrittmacher versehenen Tieren zeigten eine fast 6fache Erhöhung an Histon-assoziierten DNA-Fragmenten im Vergleich zu Extrakten von Kontroll-Myocyten (Fig.2c).

### Beispiel 4:

### Der p35- und ICAD-Gentransfer hemmen eine erhöhte Caspase-3-Aktivität und die DNA-Fragmentierung in vitro und in vivo ausreichend

Zur Manipulation des Caspase-3-aktivierten DNA-Abbau-Signalwegs als letztem Schritt in dem Prozeß des myokardialen Zelltods wurden Adenovirus-Konstrukte für p35 als einem potenten Caspase-3-Inhibitor (Ad-p35) und ICAD (Ad-ICAD) als einem "Scavenger"-Molekül für aktivierte CAD erzeugt. Die Adenovirus-Konstrukte waren so strukturiert, daß sie' das Transgen und, zur Kontrolle ausreichender Expression, außerdem GFP codierten (Ad-GFP). Außerdem waren beide Transgene zur Immunfärbung mit einem Epitop-"Tag" versehen. Vor der Bestimmung der funktionalen Folgen der Adenovirus-Infektion in insuffizientem Myokard wurde die Expression der Transgene in TNFα-stimulierten apoptotischen ventrikulären Myocyten untersucht (Fig.3a,b). Adulte Kardiomyocyten wurden mit Adenoviren mit einer Multiplizität der Infektion (moi) von 80 pfu/Zelle infiziert, von der bereits gezeigt werden konnte, daß dadurch in praktisch 100% der ventrikulären Myocyten eine optimale Expression des Transgens erreicht wird. 48 Stunden nach Infektion mit Ad-p35 bzw. Ad-ICAD exprimierten Extrakte von Myocyten signifikante Proteinspiegel, was durch Immunfärbung bestimmt wurde. Die Caspase-3-Aktivität und DNA-Fragmentierung wurden durch Behandlung mit TNFα in vitro stimuliert (Fig.3a,b). Die adenovirale Expression von p35 unterdrückte in ventrikulären Kaninchen-Kardiomyocyten in vitro die TNFα-stimulierte Caspase-3-Aktivitäten auf Basalniveau. Interessanterweise ergab die Überexpression von ICAD eine DNA/Histon-Bildung unter die Kontrollwerte (Fig.3a,b). Die Infektion von Kardiomyocyten mit rekombinantem Kontroll-Adenovirus (Ad-GFP) hatte in vitro und in vivo keinen Einfluß auf die Apoptose-Parameter bei den Myocyten (Fig.3a,b,c,d). Wie in Fig.3 und c dargestellt zeigten isolierte Myocyten von insuffizientem Myokard im Vergleich zu gesunden Myocyten eine signifikante Erhöhung der Caspase-3-vermittelten Induktion der DNA-Fragmentierung (38 ± 6 aktive Einheiten versus 12 ± 3 aktive Einheiten / 5,5fache Erhöhung der DNA/Histon-Bildung, n=5 in jeder Gruppe, p < 0,005). Nach Verabreichung der beiden Adenovirus-Transgene in vivo und anschließender Isolierung der ventrikulären Myocyten von dem myokardialen Zielbereich zeigten fast 50% der Zellen reproduzierbar eine positive GFP-Färbung. In diesen Zellen blockierte ICAD wirksam die Caspase-3-induzierte CAD-Aktivität, p35 hemmte die Enzymaktivität partiell. Kontrollinfektionen zeigten keine 'Auswirkung (Fig.3c,d).

### Beispiel 5:

### Der Adenovirus-Gentransfer in den Herzmuskel mit Insuffizienz ist wirksam

Gewebeschnitte eines repräsentativen Kaninchenherzens drei Tage nach Gentransfer von Ad-βGal (10⁹ - 10¹⁰ pfu) sind in Fig.4a gezeigt. Der Gentransfer zeigte ein Maximum der Genexpression 6 Tage nach Infektion, woran sich in den darauffolgenden Wochen ein allmählicher Abfall der Expression anschloß. Nach vier Wochen war weniger als 1% des Myokard infiziert. Zwei Drittel des Myokard der linken Kammer zeigten reproduzierbar eine Expression des Transgens (Fig.4a). Fluoreszenz-Aufnahmen makroskopischer Abschnitte eines mit dem bicistronischen Ad-ICAD infizierten Herzens ergaben eine deutliche Transgen-Expression über das gesamte manipulierte Myokard und die Immunfärbung des Epitop-"Tag" des Transgens zeigte im gleichen Bereich Signale (Fig.5a,b). Nach Infektion mit 10⁹ - 10¹⁰ pfu Ad-ICAD in vivo zeigten fast 50% der isolierten ventrikulären Kardiomyocyten grüne Fluoreszenz und eine positive Immunfärbung (Fig.5c,d). p35 (als Baculovirus-Protein) zeigte bei genauso eingestellten viralen Titern in den eukaryotischen Zellen geringere Expressionsspiegel als ICAD, was sich aus dem Immunblot von Fig.4b ergibt.

### Beispiel 6:

### Die Blockierung des Caspase-3-aktivierten Apoptose-Mechanismus verbessert die Kontraktionskraft

Zur Beurteilung der funktionellen Auswirkungen von ICAD hinsichtlich der Verhinderung des Fortschreitens der Herzinsuffizienz als Inhibitor des Schlüssel-Schritts im myokardialen Zelltod wurden echokardiographische und hämodynamische Parameter im Anschluß an den Adenovirus-vermittelten transkoronaren Gentransfer gemessen. Vor der Implantation des Schrittmachers wurden die Kaninchen mittels Gentransfer behandelt. Echokardiographische und hämodynamische Parameter wurden nach einem Schrittmacherzeitraum von 7 bzw. 15 Tagen (mit abgeschaltetem Schrittmacher) aufgezeichnet. Mit Ad-GFP infizierte Kaninchen dienten als Kontrollherzen. Fig.6a zeigt die Mittelwerte des "fractional shortening" des infizierten Bereichs für Ad-GFP und Ad-ICAD. Eine klare signifikante Erhöhung trat bei Ad-ICAD-behandelten Tieren ein, bei den mit Viren behandelten Kontrolltieren trat jedoch keine Wirkung ein (15 ± 0,9% vs. 22,3 ± 1,1%, n=6 in jeder Gruppe, p < 0,05). Der enddiastolische Durchmesser der linken Kammer betrug bei den Ad-GFP-Tieren 18 ± 0,4 mm im Vergleich dazu betrug der Durchmesser bei den ICAD-infizierten Tieren 15 ± 0,5 mm (Fig.6b). Zur Ergänzung der echokardiographischen Information zur lokalen myokardialen Kontraktionskraft wurden der enddistolische Druck der linken Kammer und +dp/dt als Maß für die globale ventrikuläre Kontraktionskraft ermittelt. Der enddiastolische Druck in der linken Kammer von mit Adenoviren für ICAD behandelten Tieren war im Vergleich zu den Ad-GFP-infizierten Zellen in signifikantem Ausmaß wieder hergestellt (10,2 ± 0,8 mm Hg vs. 14 ± 1,4 mm Hg, n=6 in jeder Gruppe, p < 0,5) (Fig.6c,d). Die basalen +dp/dt-Werte von Ad-GFP-infizierten Tieren unterschied sich nicht von der von Ad-ICAD-infizierten Tieren. Nach Injektion von Adrenalin wurde ein Dosis-abhängiger höherer Anstieg hinsichtlich +dp/dt in den ICAD-exprimierenden Kaninchen im Vergleich zu den Kontrolltieren beobachtet.

### Beispiel 7:

Die funktionelle Rekonstitution der Kontraktionskraft wird durch eine verringerte verschlechterung der Sarkomorganisation durch Überexpression von p35 oder ICAD erreicht

Zur Untersuchung der Auswirkung der p35-Expression auf die Kontraktionsfunktion einzelner ventrikulärer Herzmuskelzellen 15 Tage nach Verabreichung des Gens in vivo wurden Zellen vom Zielbereich von mit Ad-p35 und Ad-GFP-infizierten Kontrolltieren und CHF-Tieren isoliert und 18 Stunden gezüchtet. Das Transgen exprimierende Zellen wurden über GFP-Fluoreszenz selektiert. Figur 8 (a,b,c) zeigt Laser-Elektronenrastermikroskopaufnahmen nach Phalloidin-Anfärbung zur Sichtbarmachung von polymerem Aktin in Kontroll-Herzmuskelzellen und insuffizienten Herzmuskelzellen, die aus genetisch manipulierten Herzen isoliert worden waren. Interessanterweise zeigten versagende, p35 exprimierende Herzmuskelzellen eine höher organisierte Sarkomerstruktur im Vergleich zu Kontroll-infizierten Zellen mit zerstörten Sarkomereinheiten. Der durch semiquantitative Auswertung bestimmte Grad der Sarkomerorganisation ist in Figur 8(e) gezeigt. Das Verhältnis von Muskelzellen zu gut-organisierten Sarkomeren (mehr als 2/3 des Zellbereichs) war 64% ± 1% in Kontroll-Herzmuskelzellen, 13% ± 3% in insuffizienten Ad-GFP-infizierten und 52% ± 4% in Ad-p35-infizierten Herzmuslekzellen. Die Kontraktionsamplitude wurde in insuffizienten Zellen und Kontrollzellen gemessen, die mit einer Rate von ca. 70 Kontraktionen pro Minute elektrisch stimuliert worden waren. Die Adenovirus-Infektion veränderte die Kontraktionscharakteristika der Herzmuskelzellen nicht. Wie in Figur 8d dargestellt zeigten versagende, p35 exprimierende Muskelzellen ein signifikant erhöhtes "fractional shortening" im vergleich zu äquivalenten mit Ad-GFP infizierten Muskelzellen (4,3 ± 0,4% vs. 1,8 ± 0,2%; n=40 in jeder Gruppe). Diese Verbesserung der Kontraktionskraft konnte auch nach Isoprenalin-Stimulation beobachtet werden und die EC₅₀ der Dosis-Antwort-Kurve war zu höheren Werten verschoben, die denen gesunder Zellen ähnelten (Figur 8d). Diese Ergebnisse zeigen, daß p35 die Sarkomerorganisation wiederherstellt und dadurch wiederum die Kontraktionsfähigkeit versagender Herzmuskelzellen wiederhergestellt wird.

Ferner wurde die Kontraktionsamplitude in insuffizienten Zellen und Kontrollzellen gemessen, die mit einer Rate von ca. 70 Kontraktionen pro Minute elektrisch stimuliert worden waren. Wie in Figur 10 dargestellt, zeigten versagende, ICAD-exprimierende Herzmuskelzellen ein signifikant erhöhtes "factional shortening" im Vergleich zu äquivalenten mit Ad-GFP infizierten Muskelzellen nach Isoprenalin-Stimulation (Verbesserung von 3,8 ± 0,9 % auf 9,5 ± 1,16 %), während die basalen Werte nicht unterschiedlich waren (1.8 ± 0, 33 % vs. 1,75 ± 0,33 %). Diese Ergebnisse zeigen, daß auch ICAD die Kontraktionsfähigkeit versagender Herzmuskelzellen wiederherstellt.

### Beispiel 8:

### Mikroinjektion von aktivierter Caspase-3 in isolierte ventrikuläre Herzmuskelzellen

Um untersuchen zu können ob die Aktivierung von Caspase-3 für die Induktion der Verschlechterung der Sarkomerorganisation ausreicht, wurde das aktivierte Enzym in das Zytoplasma von adulten ventrikulären gesunden Herzmuskelzellen injiziert. Positive Zellen (ca. 10 - 20%) wurden mittels FITC-Fluoreszenz identifiziert. Die morphologischen Merkmale der Herzmuskelzellen nach Mikroinjektion von aktivierter Caspase-3 (4 ng/*µL und 20 ng*/µL) im Vergleich zu Kontroll-injizierten Zellen sind in Figur 9(a+b) gezeigt. Die Behandlung der Muskelzellen mit Caspase-3 (CPP-32) bewirkte eine schnelle, konzentrationsabhängige Zerstörung der geraden und gestreiften Muskelbündel (Figur 9b, linke und rechte Abbildung). "shortening"-Experimente mit Einzelzellen (mikroinjizierte Herzmuskelzellen) zeigten eine Caspase-3-vermittelte Erniedrigung der basalen und Isoprenalin-stimulierten Kontraktion (9,8% vs. 4,3% [10⁻⁸ iso]; n=30 in jeder Gruppe). Diese Effekte wurden durch Vorinkubation der Muskelzellen mit DEVD-fmk blockiert (Figur 9c).

## Patentansprüche

1. Screeningverfahren zur Identifizierung einer Verbindung, die als Caspase-3-oder CAD-Inhibitor die Kontraktionskraft des Herzens bei der Prävention oder Behandlung von Herzinsuffizienz verbessern kann, wobei das Verfahren umfasst:
(a) das Inkontaktbringen einer Testverbindung mit Caspase-3 oder CAD bzw. mit dem Caspase-3 oder CAD kodierenden Gen,
(b) die Bestimmung der noch verbleibenden Caspase-3- oder CAD-Aktivität bzw. der Erniedrigung der Genexpression,
(c) Auswahl der Testverbindung, wenn diese die Caspase-3- oder CAD-Aktivität oder Caspase-3- oder CAD-Expression erniedrigt oder hemmt als potentieller Inhibitor, sowie
(d) Bestimmung einer möglichen Kontraktionskraftverbesserung bei einem Herzen eines Tiermodells oder Herzmuskelzellen nach Verabreichung des potentiellen Inhibitors, und
(e) Auswahl des potentiellen Inhibitors als Verbindung, die als Caspase-3- oder CAD-Inhibitor die Kontraktionskraft des Herzens verbessern kann, wenn die Kontraktionskraft durch die Verabreichung verbessert wird.

2. Das Verfahren nach Anspruch 1, **dadurch gekennzeichnet, dass** in Schritt (d) der potentielle Inhibitor an ein Tiermodell für Herzinsuffizienz verabreicht wird.

3. Das Verfahren nach Anspruch 1, **dadurch gekennzeichnet, dass** in Schritt (d) der potentielle Inhibitor an isolierte Herzmuskelzellen verabreicht wird.

4. Das Verfahren nach einem der Ansprüche 1 bis 3, **dadurch gekennzeichnet, dass** Schritte (a) bis (c) in einem Screening mit hohem Durchsatz durchgeführt werden.

## Claims

1. Screening process for identifying a compound that is effective as an inhibitor of caspase-3 or caspase-activated deoxyribonuclease (CAD) for increasing the contractility of a heart in the treatment of cardiac insufficiency, the process comprising the following steps:
(a), bringing a test compound that is possibly suitable as an inhibitor into contact with caspase-3 or CAD or with the gene coding for caspase-3 or CAD; and
(b) determining the residual caspase-3 or CAD activity or the lowering of gene expression;
(c) identifying a test compound as a potential inhibitor when it lowers or inhibits the activity of caspase-3 or CAD or the expression of the gene coding for caspase-3 or CAD, and
(d) determining a possible increase in contractility by administration to an animal model or a heart muscle cell, and
(e) identifying a potential inhibitor as compound that is effective as an inhibitor of caspase-3 or CAD when the contractility is increased by the administration.

2. The process according to claim 1, **characterized in that** in step (d) the potential inhibitor is administered to an animal model for cardiac insufficiency.

3. The process according to claim 1, **characterized in that** in step (d) the potential inhibitor is administered to isolated heart muscle cells.

4. The process according to any one of claims 1 to 3, **characterized in that** steps (a) to (c) are carried out in a high throuput screening.

## Revendications

1. Procédé de criblage pour identifier un composé qui peut améliorer, en tant qu'inhibiteur de la Caspase-3 ou de la CAD, la force de contraction du coeur dans la prévention ou traitement de l'insuffisance cardiaque, le procédé comprenant les étapes consistant à :
(a) mettre un composé de test en contact avec la Caspase-3 ou la CAD ou avec un gène qui code la Caspase-3 ou la CAD,
(b) déterminer l'activité encore subsistante de la Caspase-3 ou de la CAD ou l'abaissement de l'expression du gène,
(c) sélectionner le composé de test lorsque ce dernier abaisse ou inhibe l'activité de la Caspase-3 ou de la CAD ou l'expression de la Caspase-3 ou de la CAD en tant qu'inhibiteur potentiel, et
(d) déterminer une éventuelle amélioration de la force de contraction du coeur d'un modèle animal ou dans de cellules musculaires cardiaques après l'administration de l'inhibiteur potentiel, et
(e) sélectionner l'inhibiteur potentiel comme composé qui peut améliorer la force de contraction du coeur en tant qu'inhibiteur de la Caspase-3 ou de la CAD, lorsque la force de contraction est améliorée par l'administration.

2. Procédé selon la revendication 1, **caractérisé en ce que** dans, l'étape (d), l'inhibiteur potentiel est administré à un modèle animal pour insuffisance cardiaque.

3. Procédé selon la revendication 1, **caractérisé en ce que**, dans l'étape (d), l'inhibiteur potentiel est administré à des cellules musculaires cardiaques isolées.

4. Procédé selon l'une des revendications 1 à 3, **caractérisé en ce que** les étapes (a) à (c) sont exécutées dans un criblage à haut débit.
